# EUROPÄISCHE PATENTSCHRIFT

(11) **EP 3 500 136 B1**
(45) Veröffentlichungstag und Bekanntmachung des Hinweises auf die Patenterteilung: **09.03.2022**
(21) Anmeldenummer: 17701434.7
(22) Anmeldetag: 13.01.2017
(51) Int. Cl.: A47C 1/034, A47C 1/0355

(54) **BESCHLAG FÜR EIN SITZMÖBELSTÜCK UND SITZMÖBELSTÜCK MIT EINEM SOLCHEN BESCHLAG**
FITTING FOR AN ITEM OF SEATING FURNITURE AND ITEM OF SEATING FURNITURE WITH SUCH A FITTING
ARMATURE POUR UN ELEMENT DE SIEGE ET ELEMENT DE SIEGE COMPRENANT UNE TELLE ARMATURE

(30) Priorität: 16.08.2016 EP 16184390
(43) Veröffentlichungstag der Anmeldung: 26.06.2019
(73) Patentinhaber: Beheermaatschappij Vermeulen Beesd B.V., 4104 BA Culemborg (NL)
(72) Erfinder: FISCHER, Matthias, 81101 Bratislava (SK)
(74) Vertreter: Witte, Weller und Partner Patentanwälte mbB Stuttgart
(86) Internationale Anmeldenummer: PCT/EP2017/050723
(87) Internationale Veröffentlichungsnummer: WO 2018/033256

(56) Entgegenhaltungen:
- EP-A1- 2 801 293
- US-A1- 2013 257 110

## Beschreibung

### ANWENDUNGSGEBIET UND STAND DER TECHNIK

Die Erfindung betrifft einen Möbelbeschlag für ein Sitzmöbelstück. Er dient der Verwendung in einem Sitzmöbelstück, insbesondere in Art eines Sessels oder Sofas. An einem solchen gattungsgemäßen Möbelbeschlag werden bestimmungsgemäß eine Möbelstückbasis, eine Sitzflächeneinheit mit Sitzfläche und eine Beinauflageneinheit mit einer Beinauflagefläche angebracht. Der Möbelbeschlag verbindet diese Komponenten miteinander und ermöglicht ihre Relativbewegung gegeneinander. Die Erfindung betrifft weiterhin auch ein Sitzmöbelstück mit einem solchen Beschlag.

Bei einem solchen gattungsgemäßen Sitzmöbel und einem hierfür vorgesehenen Möbelbeschlag ist vorgesehen, dass einerseits die Sitzflächeneinheit und andererseits die Beinauflageneinheit gegenüber der Basiseinheit verlagerbar sind. So kann ausgehend von einem Ausgangszustand, in dem die Beinauflageneinheit unter die Sitzflächeneinheit angeordnet ist, die Beinauflageneinheit nach vorne ausgefahren werden, um eine Nutzposition einzunehmen. Korrespondierend hierzu kann die Sitzflächeneinheit selbst abgesenkt werden, vorzugsweise in eine abgesenkte Position verschwenkt werden. Die Sitzfläche ist vorzugsweise nur oder überwiegend im rückwärtigen Bereich absenkbar.

Ein gattungsgemäßes Sitzmöbelstück und ein gattungsgemäßer Möbelbeschlag können darüber hinaus eine Rückenlehne bzw. einen korrespondierenden Beschlagsabschnitt aufweisen, wobei es im Zusammenhang mit der vorliegenden Erfindung hierauf nicht ankommt.

Bei einem gattungsgemäßen Möbelbeschlag ist naturgemäß der Wunsch gegeben, diesen preisgünstig herstellen zu können. Viele bekannte Sitzmöbelbeschläge, die eine mehrteilige Beinauflageneinheit aufweisen, verfügen jedoch über eine Vielzahl von Einzelteilen, die eine günstige Herstellung und Montage erschwert. Zudem ist es Wunsch bei einem gattungsgemäßen Möbelbeschlag, diesen so auszugestalten, dass er flexibel einsetzbar ist, d.h. nicht tief in die Bauweise des Sitzmöbelstücks im Übrigen eingreift. Auch ist gewünscht, dass der Möbelbeschlag wenig voluminös ist, so dass er, wenn möglich, nur bei näherer Betrachtung des Sitzmöbelstücks erkennbar ist.

Bekannte Sitzmöbelbeschläge sind vergleichsweise schwer und teuer. Insbesondere sind sie in Hinblick auf ihre Bauhöhe und Baubreite häufig nur schwer zu kaschieren, insbesondere, indem sie sich zu einem erheblichen Anteil bis in die Polsterung erstrecken, was zum einen eine dicke Polsterung erforderlich macht und zum anderen die Flexibilität der Verwendung solcher Beschläge verringert.

Aus der US 2013/257110 A1 ist ein Sitzmöbelstück mit einer Beinauflage bekannt, welches ein Sitzelement und einen demgegenüber beweglichen Zwischenträger aufweist, der seinerseitts durch Schwenkhebel geführt ist. Der Zwischenträger weist eine Schiebeführung auf, mittels derer ein Schiebeträger gegenüber dem Zwischenträger beweglich ist, wobei die Beinauflage an diesem Schiebeträger angebracht ist.

### AUFGABE UND LÖSUNG

Aufgabe der Erfindung ist es, einen preislich günstigen Möbelbeschlag zur Verfügung zu stellen, der aufgrund kleiner Baugröße eine besonders flexible Verwendung ermöglicht.

Zur Lösung dieser Aufgabe wird ein Möbelbeschlag vorgeschlagen, der einen Basisabschnitt zur Anbringung an einer auf einem Fußboden stehenden Möbelstückbasis, einen Sitzflächenabschnitt zur Anbringung einer Sitzflächeneinheit mit Sitzfläche und einen Beinauflagenabschnitt zur Anbringung einer Beinauflageneinheit mit Beinauflagefläche aufweist. Der erfindungsgemäße Möbelbeschlag wird in Anspruch 1 definiert.

Die drei genannten Abschnitte, der Basisabschnitt, der Sitzflächenabschnitt und der Beinauflagenabschnitt, sind definitionsgemäß und erfindungsgemäß in sich starre Bauteile, die mittels nachfolgend noch erläuterter Schwenklaschen und Schwenkhebel miteinander verbunden sind und die die Anbringungspunkte für die jeweiligen Einheiten, die Basiseinheit, die Sitzflächeneinheit mit Sitzfläche und die Beinauflageneinheit mit Beinauflagefläche des Sitzmöbelstücks zur Verfügung stellen. Gegenüber diesen Abschnitten bewegliche weitere Abschnitte der jeweils gleichen Einheit gelten nicht als Basisabschnitt, Sitzflächenabschnitt und Beinauflagenabschnitt im Sinne dieser Erfindung. So könnte beispielsweise bei einer besonderen Form einer Beinauflageneinheit ein ergänzender Abschnitt vorgesehen sein, der beweglich am Beinauflagenabschnitt beweglich ist, ohne im Sinne dieser Erfindung selbst Teil des Beinauflagenabschnittes des Beschlages zu sein. Die genannten Beschlagsabschnitte sowie die diese verbindenden Laschen und Hebel sind vorzugsweise als metallische Bauteile vorgesehen.

Der Sitzflächenabschnitt ist beweglich zwischen einer oberen und einer unteren Endlage am Basisabschnitt angebracht. Der Beinauflagenabschnitt ist beweglich zwischen einer Stau-Endlage, in der der Beinauflageabschnitt unter dem Sitzflächenabschnitt angeordnet ist, und einer Nutz-Endlage, in der der Beinauflageabschnitt vor dem Sitzflächenabschnitt angeordnet ist, am Sitzflächenabschnitt angebracht.

Im Zusammenhang mit dieser Erfindung beziehen sich die Begriffe "oben" und "unten" auf eine vertikale Möbelhochrichtung (Z), die durch die Ausrichtung des Basisbeschlags im vollständigen Sitzmöbel definiert ist. Die Begriffe "vorne" und "hinten" beziehen sich auf eine Möbellängsrichtung (X), die einer Geradeaus-Blickrichtung eines auf dem Sitzmöbelstück Sitzenden entspricht. Die zur Möbelhochrichtung (Z) und Möbellängsrichtung (X) orthogonale Richtung wird als Möbelquerrichtung (Y) bezeichnet.

Der Sitzflächenabschnitt ist zwischen seiner oberen und seiner unteren Endlage absenkbar, wobei insbesondere ein hinterer Teil des Sitzflächenabschnitts abgesenkt wird. Diese Absenkbarkeit beträgt vorzugsweise zwischen 2 und 7 cm.

Der Beinauflagenabschnitt ist in seiner Stau-Endlage so angeordnet, dass eine daran angebrachte Beinauflageneinheit vollständig unter einer am Sitzflächenabschnitt angebrachten Sitzflächeneinheit angeordnet ist. Wenn der Beinauflagenabschnitt in seiner Stau-Endlage angeordnet ist, ist die Beinauflageneinheit somit gleichsam versteckt. In der Nutz-Endlage des Beinauflagenabschnitts ist die Beinauflageneinheit vor der Sitzflächeneinheit angeordnet und mit dieser in etwa bündig ausgerichtet, so dass eine auf der Sitzflächeneinheit sitzende Person bequem ihre Beine auf die Beinauflageneinheit legen kann. Die Verlagerung des Beinauflagenabschnitts ist primär eine Verlagerung in Möbellängsrichtung (X), insbesondere vorzugsweise um mindestens 40 cm oder gar mindestens 50 cm in Möbellängsrichtung (X). Sie geht jedoch vorzugsweise auch mit einer Kippbewegung des Beinauflagenabschnitts um 20 bis 45° einher, damit die Beinauflageneinheit unter der Sitzflächeneinheit nach hinten deutlich abfallend und in der Nutzposition in etwa waagerecht ausgerichtet sein kann.

Der Beinauflageabschnitt ist mittels eines hinteren und eines vorderen Schwenkhebels am Sitzflächenabschnitt angebracht, wobei einer der Schwenkhebel um eine zum Sitzflächenabschnitt und diesem Schwenkhebel ortsfeste erste Schwenkachse gegenüber dem Sitzflächenabschnitt schwenkbeweglich ist und um einer zum Beinauflagenabschnitt und dem diesem Schwenkhebel ortsfeste zweite Schwenkachse gegenüber dem Beinauflagenabschnitt schwenkbeweglich ist, und der andere Schwenkhebel um eine zum Sitzflächenabschnitt und diesem Schwenkhebel ortsfeste dritte Schwenkachse gegenüber dem Sitzflächenabschnitt schwenkbeweglich ist und um einer zum Beinauflagenabschnitt und dem diesem Schwenkhebel ortsfeste vierte Schwenkachse gegenüber dem Beinauflagenabschnitt schwenkbeweglich ist.

Durch die beiden Schwenkhebel, die in Möbellängsrichtung hintereinander angeordnet sind, wird ein Bewegungspfad des Beinauflagenabschnitts gegenüber dem Sitzflächenabschnitt definiert. Die vier Schwenkachsen, von denen die erste und die dritte Schwenkachse ortsfest am Sitzflächenabschnitt angebracht sind und von denen die zweite und die vierte Schwenkachse ortsfest am Beinauflagenabschnitt angebracht sind, bilden gemeinsam ein Viereck bei Blickrichtung in Möbelquerrichtung, das im Weiteren zur Erläuterung der Besonderheit der Erfindung nochmals herangezogen wird. Die Abstände zwischen der ersten und der zweiten Schwenkachse und der dritten und der vierten Schwenkachse und/oder die Abstände zwischen zweiten und der dritten Schwenkachse und der vierten und der ersten Schwenkachse sind vorzugsweise unterschiedlich. Insbesondere können die Schwenkachsen des vorderen Schwenkhebels weiter voneinander beabstandet sein als die Schwenkachsen des hinteren Schwenkhebels, um die genannte Kippbewegung des Beinauflagenabschnitts zu realisieren.

Es ist eine Getriebeeinrichtung zur zumindest phasenweisen kinematischen Kopplung der Bewegung des Sitzflächenabschnitts gegenüber dem Basisabschnitt mit der Bewegung des Beinauflagenabschnitts gegenüber dem Sitzflächenabschnitt vorgesehen.

Diese Getriebeeinrichtung koppelt die Bewegungen des Sitzflächenabschnitts gegenüber dem Basisabschnitt sowie des Beinauflagenabschnitts gegenüber dem Sitzflächenabschnitt. Dies ist zum einen gewünscht, da die Absenkung des Sitzflächenabschnitts zur Erzielung einer bequemen Sitzposition bei ausgefahrener Beinauflageneinheit vorteilhaft ist. Zum anderen bewirkt diese Koppelung, dass die Gewichtskraft des auf der Sitzfläche Sitzenden geeignet ist, um die Beinauflageneinheit wippenartig in ihrer Nutzposition zu stabilisieren.

Es wird im Sinne der Erfindung jedoch als vorteilhaft angesehen, wenn die Bewegungen nicht immer gekoppelt sind oder zumindest phasenweise eine nur sehr schwache Koppelung besteht, wie im Weiteren noch erläutert wird.

Zusätzlich zur Koppelung des Beinauflagenabschnitts über die Getriebeeinrichtung mit dem Sitzflächenabschnitt, weist der Möbelbeschlag vorzugsweise eine Antriebseinrichtung auf, mittels derer ein erster der Schwenkhebel zum Zwecke des Überführen des Beinauflageabschnitts in die Nutz-Endlage angetrieben ist.

Die Antriebseinrichtung, die vorzugsweise sehr einfach ausgestaltet ist und lediglich einen Betätigungshebel zur manuellen Betätigung umfasst, der drehfest mit einem der Schwenkhebel, insbesondere dem vorderen Schwenkhebel, verbunden ist, bietet neben der Gewichtskraft, die über die Sitzflächeneinheit eingekoppelt werden kann, eine zweite Antriebsmöglichkeit, um die Beinauflageneinheit in seine Nutz-Endlage bzw. den hieran angebrachten Beinauflagenabschnitt in Richtung seiner Nutzposition zu überführen. Hierdurch wird es möglich, eine Übersetzung in der Getriebeeinrichtung zu wählen, die ausgehend von der Stau-Endlage des Beinauflagenabschnitts bei der Überführung in Richtung der Nutz-Endlage zunächst mit keiner oder einer nur sehr geringfügigen Absenkung des Sitzflächenabschnitts einhergeht. Der Vorteil dessen wird im Weiteren noch erläutert.

Die genannte besonders einfache Gestaltung der Antriebseinrichtung durch einen mit einem der Schwenkhebel ortsfest verbundenen Betätigungshebel stellt nicht die einzige Möglichkeit dar. Die Antriebseinrichtung kann alternativ ein Betätigungselement zur manuellen Betätigung umfassen, welches über ein Antriebsgetriebe in einen der Schwenkhebel ein Antriebsmoment einkoppelt.

Der Zweck eines solchen ergänzenden Antriebsgetriebes zwischen dem Betätigungselement bzw. Betätigungshebel einerseits und dem Schwenkhebel andererseits statt einer unmittelbaren Anbindung liegt darin, dass hierdurch phasenweise eine erleichterte Betätigung möglich ist. Das Antriebsgetriebe ist zu diesem Zweck vorzugsweise mit einem Übersetzungsverhältnis > 1 ausgebildet, wobei es sich insbesondere vorzugsweise um ein veränderliches Übersetzungsverhältnis handelt. Insbesondere von Vorteil ist es, wenn das Antriebsgetriebe ein variables Übersetzungsverhältnis aufweist, wobei es insbesondere von Vorteil ist, wenn dieses Verhältnis zu Beginn des Ausfahrens des Beinauflageabschnitts größer 3:1 ist und bei fortgesetzter Überführung zumindest phasenweise kleiner 2:1 ist.

Die erleichterte Betätigung aufgrund des Antriebsgetriebes mit einem Übersetzungsverhältnis größer 1 ist insbesondere von Vorteil, da bei der Überführung des Beinauflageabschnitts aus seiner StauEndlage in seine Nutz-Endlage die auf den Sitzflächenabschnitt wirkende Gewichtskraft vorzugsweise zu Beginn der Bewegung entkoppelt oder nahezu entkoppelt von der Bewegung des Beinauflageabschnitts ist. Die Bewegung des Beinauflageabschnitts wird also in einem solchen Falle zu Beginn nahezu ausschließlich über das Betätigungselement und damit vorzugsweise manuell eingebracht. Dies kann jedoch zu schwergängig sein, wenn auf die Übersetzung verzichtet wird. Zudem ist der Beinauflageabschnitt in der Stau-Endlage aufgrund der Federkraft und ggf. einer zusätzlichen Magnetsicherung gesichert, so auch zur Überwindung dessen die Übersetzung von Vorteil ist.

Die Veränderlichkeit der Übersetzung gestattet es, dass das Übersetzungsverhältnis dann im weiteren Verlauf der Überführung des Beinauflageabschnitts in die Nutz-Endlage geringer wird, so dass der Gesamtschwenkwinkel des Betätigungselementes nicht sehr viel größer als der des angetriebenen Schwenkhebels ist, in den das Antriebsmoment eingekoppelt wird.

Eine bevorzugte Ausgestaltung eines solchen Antriebsgetriebes sieht vor, dass der Betätigungshebel über eine Hebellasche, die schwenkbar an einem mit dem Betätigungshebel drehfesten Abschnitt und schwenkbar mit dem Schwenkhebel verbunden ist, am Schwenkhebel angebracht ist. Diese Gestaltung mit Hebellasche gestattet es auf sehr einfache Weise, eine anfänglich sehr große Übersetzung zu erzielen. Der Anbindungspunkt der Hebellasche an den Abschnitt des Schwenkhebels ist hierfür so anzuordnen, dass dieser Anbindungspunkt sich anfänglich annährend, jedoch nicht vollständig, orthogonal zur Verbindungslinie zwischen diesem Anbindungspunkt und dem zweiten Anbindungspunkt der Hebelasche am Schwenkhebel bewegt. Eine vergleichsweise große Schwenkbewegung des Betätigungshebels führt daher nur zu einer geringen Schwenkbewegung des Schwenkhebels und somit zu einer großen Übersetzung. Mit fortgesetzter Bewegung des Anbindungspunktes der Hebelasche am Schwenkhebel ändern sich die Winkelverhältnisse, so dass die Übersetzung sinkt.

Es ist auch eine elektrisch angetriebene Variante möglich, bei der die Antriebseinrichtung einen Elektromotor umfasst, der in einen der Schwenkhebel ein Antriebsmoment einkoppelt. Als ein solches über ein Getriebe angebundenes Betätigungselement bietet sich bei Sitzmöbelstücken mit Armlehnen eine solche Armlehne bzw. ein Abschnitt derselben an.

Die Getriebeeinrichtung weist vorzugsweise eine Übersetzungscharakteristik auf, der zufolge bei der Überführung des Beinauflagenabschnitts aus der Stau-Endlage in die Nutz-Endlage in einer Bewegungsphase ausgehend von der Stau-Endlage, in der der erste Schwenkhebel 50% seines Weges zwischen der Stau-Endlage und der Nutz-Endlage des Beinauflageabschnitts zurücklegt, der Sitzflächenabschnitt weniger als 50 seines Weges zwischen der oberen Endlage und der unteren Endlage zurücklegt, und in einer sich anschließenden Bewegungsphase bis zur Nutz-Endlage, in der der erste Schwenkhebel weitere 50% seines Weges zwischen der Stau-Endlage und der Nutz-Endlage des Beinauflageabschnitts zurücklegt, der Sitzflächenabschnitt mehr als 50% seines Weges zwischen der oberen Endlage und der unteren Endlage zurücklegt.

Diese Übersetzungscharakteristik bedeutet, dass die erste Hälfte des Weges des Beinauflagenabschnitts in Richtung der Nutz-Endlage mit einer vergleichsweise geringen Bewegung des Sitzflächenabschnitts einhergeht. Dieser legt weniger als 50% seines Gesamtweges in dieser ersten Phase der ersten Halbstrecke des Weges des Beinauflagenabschnitts zurück, wobei diese weniger als 50% auf die Gesamtlänge des Pfades bezogen sind, entlang dessen der Sitzflächenabschnitt zwischen seiner oberen und seiner unteren Endlage beweglich ist. Vorzugsweise legt der Sitzflächenabschnitt einen noch deutlich geringeren Anteil seines Gesamtweges zurück, insbesondere weniger als 25%, insbesondere vorzugsweise weniger als 15%.

Dies bedeutet, dass ausgehend von der Stauposition der Beinauflageneinheit diese vergleichsweise weit in Richtung ihrer Nutzposition ausgefahren wird, ohne dass derweil die Sitzfläche relevant abgesenkt wird. Diese Entkoppelung oder nur schwache Koppelung der Bewegungen führt dazu, dass in dieser ersten Bewegungsphase, die beispielsweise über den genannten Betätigungshebel manuell eingeleitet wird, die Gewichtskraft des auf dem Sitzmöbelstück Sitzenden noch keine große Rolle für das Ausfahren der Beinauflageeinheit spielt. Demzufolge kann das Ausfahren sehr gut dosiert werden und es besteht nicht die Gefahr, dass die Beinauflageneinheit in schmerzhafter Weise dem auf dem Sitzmöbelstück Sitzenden gegen die Unterschenkel schlägt. Mit der genannten Übersetzungscharakteristik geht auch einher, dass gegen Ende der Bewegung und somit beim Einreichen der Nutz-Endlage des Beinauflagenabschnitts die Gewichtskraft des auf dem Sitzmöbelstück Sitzenden stärker auf die Beinauflageneinheit bzw. den Beinauflagenabschnitt wirkt und somit in der Nutzposition der Beinauflageneinheit diese sehr vorteilhaft durch die Gewichtskraft des auf dem Sitzmöbelstück Sitzenden in ihrer Position stabilisiert wird.

Auch für den Vorgang der Rücküberführung der Beinauflageneinheit mit Beinauflagenabschnitt bis in die Stauposition ist diese Charakteristik von Vorteil: Das Einfahren der Beinauflageeinheit und damit das Anheben auch der Sitzflächeneinheit und des Gewichtes des Benutzers wird über die Beinmuskulatur bewirkt, was dem Benutzer bei ausgestreckten Beinen leicht fällt. Sobald der Beinauflageabschnitt um 50% in Richtung seiner Stauendlage eingeschwenkt wurde, wurde auch der überwiegende Teil (>50%) der mechanischen Arbeit zum Anheben der Sitzflächeneinheit bereits geleistet, so dass anschließend keine starke Neigung des Gesamtsystems mehr verbleibt, aufgrund der Gewichtskraft des auf dem Sitzmöbelstück Sitzenden zurück in die Nutzposition zu gelangen. Subjektiv leistet das Sitzmöbelstück keine Gegenwehr gegen das Einschwenken der Beinauflageneinheit.

Vorzugsweise weist die Getriebeeinrichtung eine Übersetzungscharakteristik auf, der zufolge bei der Überführung der Beinauflage aus der Stau-Endlage in die Nutz-Endlage in einer Bewegungsphase ausgehend von der Stau-Endlage, in Nutz-Endlage der erste Schwenkhebel 33% seines Weges zwischen der Stau-Endlage und der Nutzstellung des Beinauflageabschnitts zurücklegt, der Sitzflächenabschnitt weniger als 10%, vorzugsweise weniger als 5%, seines Weges zwischen der oberen Endlage und der unteren Endlage zurücklegt.

In diesem ersten Drittel der Bewegung des Schwenkhebels findet somit vorzugsweise kaum eine bzw. gar keine Bewegung des Sitzflächenabschnitts statt. Bein Einschwenken des Beinauflageabschnitts in Richtung seiner Stau-Endlage reicht nach etwa 70% des Schwenkwinkels daher üblicherweise die kinetische Energie der Beinauflageeinheit, um den Beinauflageabschnitt bis in seine Endlage weiterzubewegen.

Vorzugsweise sind jeweils nur ein vorderer und ein hinterer Schwenkhebel vorgesehen, wobei diese Schwenkhebel jeweils vorzugsweise schmaler als 3 cm sind.

Die Verwendung von jeweils nur einem vorderen und einem hinteren Hebel ist deshalb von Vorteil, da bei einem erfindungsgemäßen Sitzmöbelstück üblicherweise geboten ist, dass die Beinauflagenfläche durch eine im Weiteren noch beschriebene spaltartige Unterbrechung einen Aufnahmebereich für die Schwenkhebel in der Stau-Endlage bietet. Durch die Verwendung nur eines vorderen und eines hinteren Schwenkhebels, die bezogen auf die Möbelquerrichtung übereinstimmend angeordnet sind, reicht ein schmaler Spalt in der Beinauflagenfläche. Die Breite der Schwenkhebel in jenem Mittelabschnitt, in dem sie bestimmungsgemäß durch die Beinauflagenfläche hindurchgeführt werden, beträgt maximal die genannten 3 cm, vorzugsweise sind es 2 cm oder weniger. Im Bereich der Achsen, mittels derer die Schwenkhebel am Sitzflächenabschnitt einerseits und am Beinauflagenabschnitt andererseits angebracht sind, verfügen die Schwenkhebel vorzugsweise über fest mit den Schwenkhebeln verbundene Schwenkwellen mit einer Länge von mindestens 5 cm, vorzugsweise mindestens 8 cm, jedoch insbesondere vorzugsweise maximal 15 cm, durch die durch nicht zentrische Kraftbeaufschlagung in den Beinauflagenabschnitt auftretende Momente in den Sitzflächenabschnitt abgeleitet werden können. Diese Schwenkwellen können als Stangen- oder Rohrabschnitte ausgebildet sein, die durch eine Schweiß- oder Schraubverbindung Teil der Schwenkhebel sind. Eine preisgünstigere Gestaltung ist erzielbar, wenn die Schwenkwellen durch eine Umformbearbeitung mit den Schwenkhebeln verbunden sind. So kann insbesondere der Schwenkhebel durch zwei Flächen gebildet werden, die im Mittelabschnitt aneinander anliegen und dort per Schraub-, Löt- oder Schweißverbindung verbunden sind, die jedoch endseitig gabelartig umgebogen sind, um dadurch eine Schwenkwelle zu bilden.

Alternativ zu einer Gestaltung mit jeweils nur einem vorderen und hinteren Schwenkhebel sind jedoch auch Gestaltungen denkbar, bei denen diese jeweils doppelt in Möbelquerrichtung voneinander beabstandet vorgesehen sind. Hierbei wird jedoch bevorzugt, wenn der Abstand der jeweiligen Außenflächen der Schwenkhebel 15 cm nicht übersteigt, um eine versteckte zentrische Anbringung des Möbelbeschlages zu gestatten. Die Verwendung von insbesondere jeweils zwei vorderen und hinteren Schwenkhebeln gestattet eine dünnere Ausgestaltung, beispielsweise mit einer Erstreckung in Möbelquerrichtung von nur 0,5 cm. In diesem Fall sind in der Beinauflagefläche vorzugsweise zwei getrennte schlitzartige Unterbrechungen vorgesehen.

Der Sitzflächenabschnitt ist vorzugsweise mittels mindestens einer Schwenklasche an den Basisabschnitt angebunden, wobei vorzugsweise zwei Schwenklaschen mit jeweils unterschiedlichen Drehachsen hierfür vorgesehen sind, wie im Weiteren noch erläutert wird.

Weiterhin ist vorzugsweise an einem der Schwenkhebel, vorzugsweise am hinteren Schwenkhebel, eine Antriebslasche schwenkbar angelenkt und die Getriebeeinrichtung ist zwischen der Schwenklasche und der Antriebslasche vorgesehen und verfügt über eine Steuerspur, entlang derer ein von der Schwenklasche angetriebenes Führungselement beweglich ist.

Die mindestens eine Schwenklasche, die den Basisabschnitt mit dem Sitzflächenabschnitt verbindet und schwenkbeweglich an beiden angebracht ist, wird demzufolge vorzugsweise genutzt, um die durch die Gewichtskraft des Sitzenden verursachte Verlagerung des Sitzflächenabschnitts auf den Beinauflagenabschnitt zu übertragen. Die Verwendung einer Steuerspur mit entlang der Steuerspur beweglichem Führungselement gestattet es dabei, die Wirkung im Sinne der oben beschriebenen Nichtlinearität flexibel ausgestalten zu können. Insbesondere gestattet es eine solche Getriebeeinrichtung mit Steuerspur, eine anfängliche Beweglichkeit des Beinauflagenabschnitts bei stillstehendem Sitzflächenabschnitt gegenüber dem Basisabschnitt zu ermöglichen.

Bei einer bevorzugten Ausgestaltung ist das Führungselement ortsfest an der Schwenklasche vorgesehen und befindet sich im Eingriff mit der Steuerspur, die ihrerseits eine gekrümmte Formgebung aufweist. Dabei ist vorzugsweise die Führungsspur an der Antriebslasche vorgesehen und diese insbesondere vorzugsweise mittels einer Zwischenlasche an der Sitzflächeneinheit angebracht. Diese Bauweise hat sich als besonders einfach herausgestellt und geht mit sehr wenig beweglichen Bauteilen einher. Die auf einen der Schwenkhebel wirkende Antriebslasche ist gleichzeitig Träger der Steuerspur und über eine Zwischenlasche an der Sitzflächeneinheit angebracht. In die hierbei gekrümmte Steuerspur greift das Führungselement ein, welches vorzugsweise ortsfest an der genannten Schwenklasche vorgesehen ist.

Eine Gestaltung mit gekrümmter Steuerspur ist nicht alternativlos. Eine lineare Steuerspur ist ebenfalls möglich, wobei in diesem Falle vorzugsweise eine weitere Zwischenlasche vorgesehen ist, die schwenkbar an der Schwenklasche zwischen Basisabschnitt und Sitzflächenabschnitt angebracht ist und mit dem Führungselement versehen ist.

Die Antriebseinrichtung, also insbesondere der Betätigungshebel zur manuellen Betätigung, wirkt vorzugsweise auf den mittels der ersten und der zweiten Schwenkachse angebundenen Schwenkhebel, der vorzugsweise den vorderen Schwenkhebel bildet.

Dies ist insbesondere bei einer Gestaltung wünschenswert, bei der in der Stauendlage eine Verbindungslinie zwischen der ersten Schwenkachse und der zweiten Schwenkachse mit einer Verbindungslinie zwischen der zweiten Schwenkachse und der vierten Schwenkachse einen Winkel < 20° einschließt oder sogar sich die zweite Schwenkachse in einer Übertotpunkt-Lage befindet, so dass ein durch die Lage der ersten, zweiten, dritten und vierten Schwenkachse definiertes Viereck ein konkaves Viereck bildet, dessen zweite Schwenkachse die konkave Ecke bildet.

Diese Gestaltung mit einer sehr gestreckten Ausrichtung der beiden genannten Verbindungslinien, die durch die Ausrichtung eines der Schwenkhebel und des Beinauflageabschnitts definiert ist, oder sogar einer Übertotpunktlage der zweiten Schwenkachse gestattet es in besonders vorteilhafter Weise, einen vergleichsweise großen Schwenkwinkel eines der Schwenkhebel zu zwischen Stau-Endlage und Nutz-Endlage zu realisieren, insbesondere vorzugsweise von mindestens 130°, so dass hierdurch die unmittelbare Verbindung des Sitzflächenabschnitts und des Beinauflagenabschnitts über die Schwenkhebel ermöglicht wird. Würde ein kleinerer Schwenkwinkel unter Meidung der genannten gestreckten Lage bzw. der Übertotpunktlage angestrebt, so müssten die Schwenkhebel nochmals länger ausgestaltet sein, um ein ausreichendes Ausfahren des Beinauflagenabschnittes zu ermöglichen. Dies jedoch ist bei den meisten Sitzmöbelstücken aufgrund des zur Verfügung stehenden Bauraums und insbesondere aufgrund der begrenzten Höhe der Sitzfläche über einem Untergrund kaum zu realisieren. Dass diese Nahezu-Totpunktlage oder sogar die Übertotpunktlage möglich wird, hängt auch damit zusammen, dass die genannte Antriebseinrichtung vorgesehen ist, mittels derer einer der Schwenkhebel insbesondere manuell verschwenkt werden kann. Die üblicherweise mit der Annäherung an einer Totpunktlage einhergehende mechanische Instabilität aufgrund des Wegfalls der statischen Bestimmtheit wird durch das unmittelbare Antreiben einer der Schwenkhebel überwunden.

Bei einem erfindungsgemäßen Beschlag wird es als vorteilhaft angesehen, wenn die sitzabschnittsseitigen Schwenkachsen der Schwenkhebel zwischen Sitzflächenabschnitt und Beinauflagenabschnitt und oder der Schwenklaschen zwischen Sitzflächenabschnitt und Basisabschnitt unterhalb der Sitzfläche der Sitzflächeneinheit vorgesehen sind, vorzugsweise deutlich darunter, wobei dies mindestens 2 cm, vorzugsweise mindestens 4 cm bedeutet. Bezogen auf den Sitzflächenabschnitt des Möbelbeschlages ist es von Vorteil, wenn die erste und dritte Schwenkachse unterhalb von Anbringungspunkten vorgesehen sind, mittels derer eine Grundplatte oder ein Grundrahmen der Sitzflächeneinheit mit dem Sitzflächenabschnitt des Möbelbeschlages verbunden wird.

Der Schwenkwinkel, den einer der Schwenkhebel zwischen der Stau-Endlage und der Nutz-Endlage des Beinauflageabschnittes überstreicht, beträgt vorzugsweise mindestens 130°, insbesondere vorzugsweise mindestens 140°. Der Schwenkwinkel, den der andere Schwenkhebel zwischen der StauEndlage und der Nutz-Endlage des Beinauflageabschnittes überstreicht, beträgt vorzugsweise mindestens 110°, insbesondere vorzugsweise mindestens 120°. Vorzugsweise wird der größere Schwenkwinkel vom vorderen Schwenkhebel überstrichen.

Zur beweglichen Anbringung des Sitzflächenabschnittes an den Basisabschnitt sind vorzugsweise zwei Schwenklaschen vorgesehen, wobei eine erste der Schwenklaschen um eine zur ersten Schwenklasche und zum Sitzflächenabschnitt ortsfeste fünfte Schwenkachse schwenkbar am Sitzflächenabschnitt angebracht ist und um eine zur ersten Schwenklasche und zum Basisabschnitt ortsfeste sechste Schwenkachse schwenkbar am Basisabschnitt angebracht ist, und eine zweite der Schwenklaschen um eine von der fünften Schwenkachse abweichende und zur zweiten Schwenklasche und zum Sitzflächenabschnitt ortsfeste siebente Schwenkachse schwenkbar am Sitzflächenabschnitt angebracht ist und um eine von der sechsten Schwenkachse abweichende und zur zweiten Schwenklasche und zum Basisabschnitt ortsfeste achte Schwenkachse schwenkbar am Basisabschnitt angebracht ist.

Dabei sind vorzugsweise jeweils zwei erste Schwenklaschen und zweite Schwenklaschen vorgesehen, wobei die jeweils zwei Schwenklaschen um nicht mehr als 15 cm in einer Möbelquerrichtung voneinander beabstandet sind. Ebenso wie andere Komponenten sorgen sie damit dafür, dass die Gesamtbreite des Beschlages, ohne Berücksichtigung einer etwaigen Torsionsstange zur Anbindung der Antriebseinrichtung, kaum größer 15 cm ist. Bei einer Gestaltung mit zwei ersten Schwenklaschen oder zwei zweiten Schwenklaschen, ist es von Vorteil, wenn die Schwenklaschen mindestens eines der Schwenklaschenpaare miteinander verbunden sind, vorzugsweise durch eine Verbindung im Bereich einer der Schwenkachsen der Schwenklaschen.

Die Anbindung eines Sitzflächenabschnitts am Basisabschnitt mittels zweier Schwenklaschen mit jeweils unterschiedlichen Schwenkachsen ist für sich genommen bei anderen Möbelstücken bereits realisiert worden. Besonders bevorzugt ist beim vorliegenden Möbelbeschlag die Gestaltung, bei der paarig jeweils zwei erste und zwei zweite Schwenklaschen vorgesehen sind, die jeweils um die gleichen Schwenkachsen verschwenkbar sind, wobei sie durch einen Abstand von maximal 15 cm zentrisch unter der Sitzflächeneinheit angeordnet sein können und somit von außen kaum zu erkennen sind und insbesondere auch bei Sitzmöbelstücken ohne Armlehnen eine solche Gestaltung gestatten.

Die fünfte und die siebente Schwenkachse sind voneinander vorzugsweise um nicht mehr als 25 cm, insbesondere vorzugsweise um nicht mehr als 15 cm, voneinander beabstandet und vorzugsweise in einem rückwärtigen Bereich des Möbelbeschlages vorgesehen.

Die Schwenkachsen der Schwenklaschen derart nah einander vorzusehen gestattet es, diese weit hinten am Möbelbeschlag anzubringen, wo vergleichsweise viel Bauraum zur Verfügung steht. In einem Bereich weiter vorne kann somit auf die Anbringung von Schwenklaschen verzichtet werden, so dass die Sichtbarkeit des Möbelbeschlages von außen weiter verringert wird.

Insbesondere um die Anordnung der Schwenklaschen im hinteren Bereich des Möbelbeschlages zu realisieren, ist es von Vorteil, wenn die basisabschnittseitigen sechste und achte Schwenkachse höhenversetzt zueinander angeordnet sind, insbesondere in Möbelhochrichtung um mindestens 4 cm voneinander beabstandet sind.

Die erste und die zweite Schwenklasche verbinden den Sitzflächenabschnitt und den Basisabschnitt in ähnlicher Weise wie die oben beschriebenen Schwenkhebel den Sitzflächenabschnitt und den Beinauflagenabschnitt miteinander verbinden. Auch hier sind unterschiedliche Abstände zwischen den Schwenkachsen und damit auch die unterschiedlichen Winkel an den Schwenkachsen von Vorteil, um eine Kippbewegung des Sitzflächenabschnitts zu erzielen. Diese Kippbewegung ist im idealen Fall so geartet, dass nur ein rückwärtiges Ende der Sitzflächeneinheit abgesenkt wird, während ein nach vorne weisendes Ende nahezu lagestabil bleibt, so dass der Sitzflächenabschnitt gleichsam um eine virtuelle Schwenkachse schwenkt.

Bei vollständig abgesenktem Sitzflächenabschnitt und damit bei Anordnung des Beinauflageabschnitts in seiner Nutz-Endlage, schließt eine gedachte Verbindungslinie zwischen den Schwenkachsen einer der Schwenklaschen mit der Möbellängsrichtung einen Winkel <30° ein, vorzugsweise <20°. Die jeweilige Schwenklasche ist also fast horizontal ausgerichtet, was in Hinblick auf eine kompakte Bauweise des Beschlages von Vorteil ist. Insbesondere vorzugsweise sind beiden Laschen mit einer solchen Ausrichtung versehen.

Der Möbelbeschlag verfügt vorzugsweise über eine Federeinrichtung, mittels derer der Sitzflächenabschnitt und der Beinauflagenabschnitt gegeneinander federkraftbeaufschlagt werden.

Die Federeinrichtung ist durch ihre Anbringung am Beinauflagenabschnitt besonders elegant unterzubringen und muss nicht im hinsichtlich des Platzes begrenzten Bereich am Sitzflächenabschnitt unterhalb der Sitzflächeneinheit vorgesehen werden. Die Feder soll bestimmungsgemäß in etwa die Gewichtskraft der Beinauflage kompensieren, so dass diese aus ihren in der Stau-Endlage und der Nutz-Endlage gegebenen Zuständen höchster potentieller Energie nicht von selbst absinkt. Die Feder ist dementsprechend vorzugsweise derart angebracht, dass sie bei einer Überführung des Beinauflagenabschnitts aus der Stau-Endlage in die Nutz-Endlage zunächst gespannt und dann wieder entspannt wird. Es besteht demzufolge eine Umschlagsstellung, ab der die Federkraft der gespannten Feder nicht mehr in Richtung der Stau-Endlage, sondern in Richtung der Nutz-Endlage wirkt.

Dabei kann insbesondere die Federeinrichtung als Federeinrichtung mit zwei entlang einer Federrichtung linear gegeneinander beweglichen Anlenkpunkten ausgebildet sein, wobei die Federrichtung in der Haupterstreckungsrichtung der Beinauflageeinheit liegt oder mit dieser einen Winkel von weniger als 20° einschließt. Diese Haupterstreckungsebene der am Beinauflagenabschnitt anzubringenden Beinauflageeinheit mit Beinauflagefläche wird durch den Beinauflageabschnitt und die daran vorgesehenen Anbringungsstellen für die Beinauflageeinheit definiert.

Diese beschriebene Anordnung führt dazu, dass die Feder nahezu unsichtbar am Beinauflagenabschnitt versteckt werden kann. Der Beinauflagenabschnitt weist üblicherweise entsprechend der Beinauflageneinheit, die er zu tragen hat, eine längs erstreckte Form auf, durch die die Haupterstreckungsebene des Beinauflagenabschnitts definiert wird. Die Feder erstreckt sich etwa in dieser Ebene.

Darüber hinaus hat die Anordnung der Federeinrichtung an der Beinauflageneinheit auch den Vorteil, dass bei einer Möbelserie mit unterschiedlichen Beinauflagen in Abhängigkeit des jeweiligen Gewichtes der Beinauflage unterschiedliche Federn in Vormontageeinheiten der Beinauflageneinheiten vorgesehen werden können. Eine schwerere Beinauflage kann somit mit einer stärkeren Federeinrichtung versehen werden.

Die Federeinrichtung ist vorzugsweise an zwei Anlenkpunkten an unterschiedlichen Komponenten des Möbelbeschlags angebunden. Dabei ist ein erster Anlenkpunkt am Beinauflagenabschnitt vorgesehen, während ein zweiter Anlenkpunkt an einem der Schwenkhebel vorgesehen ist oder an einem entlang einer Federspur verschieblichen Schieber vorgesehen ist, der über einer Federlasche mit einem der Schwenkhebel gekoppelt ist.

Insbesondere die Verwendung mit einer Federspur mit daran angeordnetem Schieber ist von Vorteil, da hierdurch relativ zum Beinauflageabschnitt eine rein lineare Bewegung des zweiten Anlenkpunktes erzielt wird, so dass die Feder in einem sehr kleinen Bauraum des Beinauflagenabschnitts unterzubringen ist.

Vorteilhaft kann es auch sein, wenn der Möbelbeschlag eine weitere Federanordnung aufweist, die eine Feder umfasst, welche den Sitzflächenabschnitt in jenem Zustand, in dem der Beinauflageabschnitt seine Nutz-Endlage erreicht hat, nach oben oder nach unten kraftbeaufschlagt. Es kann sich um eine Zugfeder oder um eine Druckfeder handeln. Je nach Ausgestaltung bewirkt diese Feder im abgesenkten Zustand des Sitzflächenabschnitts und somit bei Anordnung des Beinauflageabschnitts in der Nutz-Endlage eine Kraftbeaufschlagung des Sitzflächenabschnitts nach oben oder unten und unterstützt oder erschwert die Rücküberführung des Beinauflageabschnitts in die Stau-Endlage.

Durch passende Auslegung dieser Feder kann der Beschlag somit auf einfache Art und Weise auf Menschen verschiedener Größe angepasst werden. Hierbei kann es sich um eine im Betrieb unveränderliche Anpassung handeln, die beispielsweise nur für den Vertrieb des Beschlages für verschiedene Ländern entsprechend der landestypischen Physiognomie erfolgt. Vorzugsweise jedoch ist die Federanordnung vom Endanwender manuell verstellbar, so dass die Kraft, mit der der Sitzflächenabschnitt bei Erreichen der Nutz-Endlage des Beinauflageabschnitts durch die Feder beaufschlagt wird, einstellbar ist. Der Endanwender kann somit passend für seine Statur den Beschlag so anpassen, dass der Beinauflageabschnitt bei Nutzung stabil in seiner Lage verharrt, bis der Nutzer mit sanftem Druck die Überführung in die Stau-Endlage einleitet.

Die Erfindung betrifft weiterhin auch ein Sitzmöbelstück in Art eines Sessels oder Sofas mit einem Möbelbeschlag vorbeschriebener Art.

Dieses Sitzmöbelstück verfügt über eine Basiseinheit, an der der Basisabschnitt des Möbelbeschlages angebracht ist. Diese Basiseinheit kann mindestens einen Möbelstückfuß zum Aufstellen des Möbelstücks aufweisen, an dem ortsfest der Basisabschnitt angebracht ist. Alternativ kann die Basiseinheit als drehbare Basiseinheit ausgebildet sein mit mindestens einem Möbelstückfuß zum Aufstellen des Möbelstücks, an dem um eine Vertikalachse drehbar ein Drehteil vorgesehen ist, an dem wiederum ortsfest der Basisabschnitts des Möbelbeschlages angebracht ist.

Das Sitzmöbelstück verfügt weiterhin über eine Sitzflächeneinheit, die eine Sitzfläche des Sitzmöbelstücks aufweist und die an dem Sitzflächenabschnitt des Möbelbeschlages angebracht ist, sowie über eine Beinauflageneinheit, die eine Beinauflagenfläche aufweist und die am Beinauflagenabschnitt des Möbelbeschlages angebracht ist.

Die Sitzflächeneinheit und/oder die Beinauflageneinheit sind vorzugsweise gepolstert, wie es bei Sofas und Sesseln meist der Fall ist. Grundsätzlich gestattet ein erfindungsgemäßer Möbelbeschlag aufgrund seiner sehr kompakten Baugröße jedoch auch die Verwendung an Sitzmöbelstücken mit nur dünner Polsterung oder gar an Möbelstücken mit nicht gepolsterten, sondern anderweitig nachgiebigen Sitz- und Rückenflächen, wie sie beispielsweise als Büromöbel mit nachgiebigen Lederflächen bekannt sind.

Der Beinauflagenabschnitt des Möbelbeschlags ist bei einer gepolsterten Beinauflageneinheit vorzugsweise zum überwiegenden Teil innerhalb eines durch den Bezug umgebenden Raums angeordnet, wobei insbesondere vorzugsweise ein Antriebshorn an der Unterseite des Beinauflagenabschnitts vorgesehen sein kann. Dieses Antriebshorn bildet vorzugsweise den unteren Abschluss des Beinauflageabschnitts. Durch eine Beabstandung von vorzugsweise mindestens 5cm, insbesondere vorzugsweise von 7 cm gegenüber der Beinauflagefläche wird erreicht, dass die in die Beinauflageeinheit eingekoppelte Gewichtskraft der Beine des Sitzenden über den am Antriebshorn angelenkten Schwenkhebel gut in Richtung der Sitzeinheit abgeleitet werden kann.

Besonders bevorzugt ist eine Gestaltung des Sitzmöbelstücks, bei der an der Sitzflächeneinheit der Möbelbeschlag von unten an einen Rahmen der Sitzflächeneinheit angeschraubt ist. Der Möbelbeschlag kann zu diesem Zweck Schraubbohrungen aufweisen.

Bei einer Gestaltung als Sofa sind vorzugsweise mindestens zwei Beschläge der vorbeschriebenen Art vorgesehen, durch die mindestens zwei Sitzflächeneinheiten und zwei Beinauflageeinheiten unabhängig voneinander gegenüber einer gemeinsamen Basiseinheit verlagerbar sind.

Bei einer besonderen Gestaltung weist die Beinauflagenfläche mindestens eine bezogen auf die Nutzstellung der Beinauflageneinheit in Richtung der Sitzflächeneinheit weisende Unterbrechung auf, in die mindestens einer der Schwenkhebel eintaucht, wenn sich die Beinauflageneinheit in ihrer StauStellung befindet.

Dabei ist ein Bereich der Beinauflagenfläche, in dem die mindestens eine zur Aufnahme des mindestens einen Schwenkhebels vorgesehene Unterbrechung vorgesehen ist, bezogen auf eine Möbelquerrichtung vorzugsweise zentrisch angeordnet und weist dabei eine Breite von maximal 15 cm auf.

Um die Beinauflageeinheit in ihrer Stauposition zu verstecken, kann es von Vorteil sein, am vorderen Ende des Sitzflächeneinheit eine nach unten gerichtete Blende vorzusehen, hinter der Stauraum zumindest für einen Vorderabschnitt der Beinauflageeinheit gegeben ist. Die Anbindung der Beinauflageeinheit an die Sitzflächeneinheit und die vorgesehenen Schwenkwinkel der Schwenkhebel sorgen dafür, dass die Beinauflageneinheit bei ihrer Überführung aus der Nutz-Position in die Stau-Position einen tiefsten Punkt überfährt und anschließend wieder ansteigt. Dies gestattet es, mit einer einfachen Blende die dahinter verschwindende Beinauflageeinheit zu kaschieren.

### KURZBESCHREIBUNG DER ZEICHNUNGEN

Weitere Vorteile und Aspekte der Erfindung ergeben sich aus den Ansprüchen und aus der nachfolgenden Beschreibung zweier bevorzugter Ausführungsbeispiele der Erfindung, die nachfolgend anhand der Figuren erläutert ist.
Fig. 1 und 2 zeigen ein Sitzmöbelstück in zwei Zuständen, nämlich einem Zustand mit Beinauflageneinheit in der Stauposition und einem Zustand mit der Beinauflageneinheit in der Nutzposition.
Fig. 3 zeigt einen Möbelbeschlag des Sitzmöbelstücks, der für die Relativverlagerbarkeit einer Sitzflächeneinheit und der Beinauflageneinheit gegenüber einer Basiseinheit des Sitzmöbelstücks verantwortlich ist.
Fig. 4A und 4B bis 7A und 7B zeigen den Möbelbeschlag in einer perspektivischen Ansicht und einer schematischen Seitenansicht in vier Zuständen, ausgehend von einem Zustand mit einem Beinauflagenabschnitt in einer Stau-Endlage und endend in einem Zustand mit dem Beinauflageabschnitt in einer Nutz-Endlage.
Fig. 8 und 9 verdeutlicht die Gesamtrelativverlagerung eines Schwenkhebels relativ zum Sitzflächenabschnitt und des Sitzflächenabschnitts relativ zum Basisabschnitt.
Fig. 10A bis 10D zeigen ein zweites Ausführungsbeispiel, welches funktional zwei relevante Änderungen gegenüber dem Ausführungsbeispiel der Fig. 1 bis 9 aufweist.

### DETAILLIERTE BESCHREIBUNG DER AUSFÜHRUNGSBEISPIELE

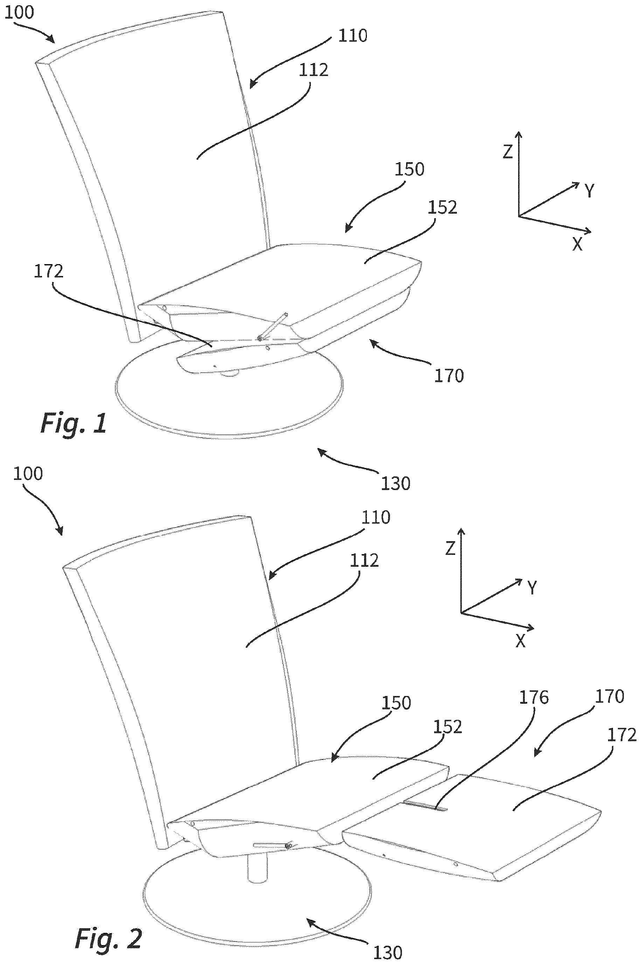

Die Fig. 1 und 2 zeigen ein erfindungsgemäßes Sitzmöbelstück 100 in Form eines Sessels in zwei Zuständen. Dieses Sitzmöbelstück 100 verfügt über eine Basiseinheit 130, eine Sitzflächeneinheit 150 mit Sitzfläche 152, einer Beinauflageneinheit 170 mit Beinauflagenfläche 172 sowie eine Rückenlehneneinheit 110 mit einer Rückenlehnenfläche 112.

Im Zustand der Fig. 1 befindet sich die Sitzflächeneinheit 150 in einer oberen Position gegenüber der als Drehfuß ausgestalteten Basiseinheit 130. Die Beinauflageneinheit 170 befindet sich bei nach oben gerichteter Beinauflagefläche 172 in einer Stauposition unterhalb der Sitzflächeneinheit 150.

Im Zustand der Fig. 2 ist die Sitzflächeneinheit 150 am hinteren Ende abgesenkt und befindet sich somit in ihrer unteren Position. Die Beinauflageneinheit 170 ist ausgefahren, so dass sie bezogen auf die Möbellängsrichtung X vor der Sitzflächeneinheit 150 angeordnet ist. Gleichzeitig ist die Beinauflageneinheit um etwa 30° gegenüber ihrer Ausrichtung der Fig. 1 frontseitig nach unten verschwenkt. Dies ist die Nutzposition der Beinauflageeinheit 170.

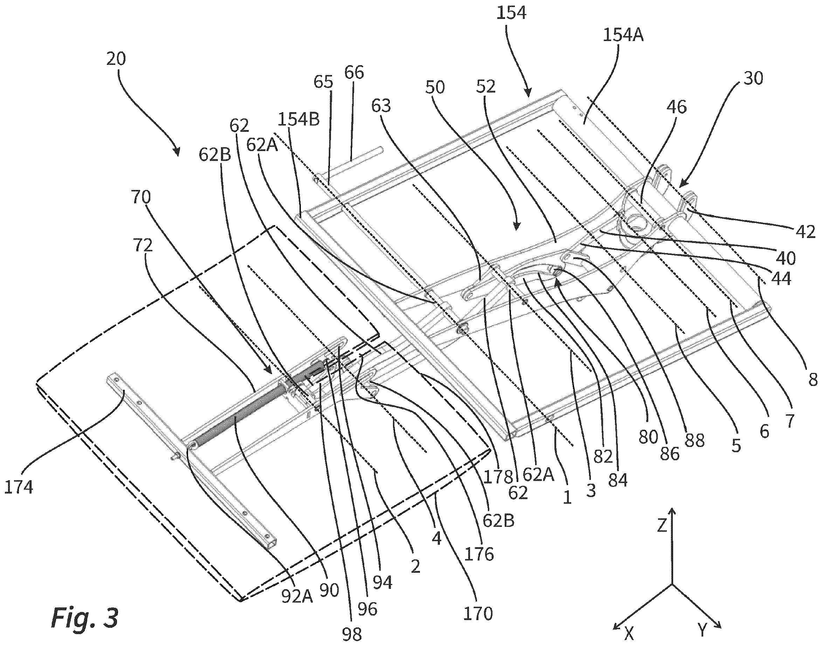

Die Relativbewegung der Sitzflächeneinheit 150 gegenüber der Basiseinheit 130 sowie der Beinauflageneinheit 170 gegenüber der Sitzflächeneinheit 150 wird über einen Möbelbeschlag 20 realisiert, der in Fig. 3 näher dargestellt ist.

Der Möbelbeschlag 20 weist als Hauptkomponenten einen Basisabschnitt 30 auf, der an der als Drehfuß ausgebildeten Basiseinheit 130 angebracht ist. Er weist weiterhin einen Sitzflächenabschnitt 50 auf, der an einem Rahmen 154 der Sitzflächeneinheit 150 angebracht ist und der über zwei voneinander um knapp 10 cm beabstandete Wangen 52 verfügt. Die dritte Hauptkomponente des Möbelbeschlages 20 ist der Beinauflagenabschnitt 70, der ebenfalls über zwei Wangen 72 verfügt, die an einem endseitigen Querrohr 174 der Beinauflageneinheit befestigt sind.

Die grundsätzliche Relativbeweglichkeit dieser Abschnitte zueinander ist wie folgt realisiert. Der Sitzflächenabschnitt 50 ist mittels zweier Schwenklaschen 40, 42 an dem Basisabschnitt 30 angebracht, wobei jeweils parallel zwei erste und zweite Schwenklaschen vorgesehen sind, die im Weiteren jedoch jeweils gemeinsam als erste bzw. die zweite Schwenklasche bezeichnet werden. Vorliegend sind die beiden hinteren Schwenklaschen 42 durch ein angeschweißtes Verbindungsrohr 46 miteinander verbunden, um hier eine erhöhte Stabilität zu erzielen. Dies könnte alternativ oder zusätzlich auch an der vorderen Schwenklasche 40 ähnlich realisiert sein.

Die erste Schwenklasche 40 ist um die Schwenkachse 5 schwenkbar am Sitzflächenabschnitt 50 angelenkt und um die Schwenkachse 6 schwenkbar am Basisabschnitt 30 angelenkt. Korrespondierend hierzu ist die zweite Schwenklasche 42 um die Schwenkachse 7 schwenkbar am Sitzflächenabschnitt 50 und um die Schwenkachse 8 schwenkbar am Basisabschnitt 30 angelenkt. Die Schwenklaschen 40, 42 definieren somit gemeinsam die Relativbeweglichkeit des Sitzflächenabschnitts 50 gegenüber des Basisabschnitt 30, wobei die jeweiligen Abstände zwischen den Achsen so gewählt sind, dass bezogen auf den Rahmen 154 die Verlagerung gegenüber dem Basisabschnitt 30 am stärksten im Bereich von dessen rückwärtigem Rohr 154A stattfindet, während das vordere Rohr 154B bei dieser Bewegung nahezu ortsfest zum Basisabschnitt 30 verbleibt.

In ähnlicher Weise wie mittels der Schwenklaschen 40, 42 ist auch der Beinauflagenabschnitt 70 am Sitzflächenabschnitt 50 angelenkt, wobei dies mittels zweier Schwenkhebel 60, 62 erfolgt, wobei diese Schwenkhebel in Möbellängsrichtung X hintereinander angeordnet sind. Der Schwenkhebel 60 bildet den vorderen Schwenkhebel. Der Schwenkhebel 62 bildet den hinteren Schwenkhebel. Beide Schwenkhebel sind an gegenüberliegenden Enden mit Schwenkwellen 60A, 60B, 62A, 62B versehen, die drehbar zwischen den Wangen 52, 72 des Sitzflächenabschnitts 50 und des Beinauflagenabschnitts 70 eingesetzt sind. Hierdurch werden die erste und die dritte Schwenkachse 1, 3 definiert, um die die Schwenkhebel 60, 62 gegenüber dem Sitzflächenabschnitt 50 verschwenkbar sind, sowie die zweite und die vierte Schwenkachse 2, 4, um die die Schwenkhebel 60, 62 schwenkbar am Beinauflagenabschnitt 70 angelenkt sind. Die Schwenkwellen 60A, 60B, 62A, 62B sind vorliegend als an Mittelbereichen der Schwenkhebel angeschweißte Rohrabschnitte ausgebildet, könnten jedoch auch durch eine umformende Biegebearbeitung oder Abkröpfung auch einstückig mit als Blechteilen ausgebildeten Mittelbereichen verbunden sein.

Zur unmittelbaren Beeinflussung des Schwenkwinkels des ersten Schwenkhebels 60 gegenüber dem Sitzflächenabschnitt 50 ist ein Betätigungshebel 66 vorgesehen, der über ein Torsionsrohr 65 drehfest mit dem vorderen Schwenkhebel 60 verbunden ist.

Weiterhin ist eine Getriebeeinrichtung 80 zur Kopplung der Schwenklasche 40 mit dem hinteren Schwenkhebel 62 vorgesehen. Diese Getriebeeinrichtung 80 umfasst einen Ausleger 44, der fest an der ersten Schwenklasche 40 angebracht ist und an dessen Ende ein Führungselement 86 vorgesehen ist. Die Getriebeeinrichtung 80 verfügt weiterhin über eine Antriebslasche 82, die an einem zum Schwenkhebel 62 ortsfesten Ausleger 63 schwenkbar angelenkt ist und die ihrerseits über eine Zwischenlasche 88 beweglich gegenüber dem Sitzflächenabschnitt 50 angebracht ist. In dieser Antriebslasche 82 ist eine Steuerspur 84 vorgesehen, innerhalb derer das bereits beschriebene Führungselement 86 am Ausleger 44 der Schwenklasche 40 eingreift.

Weiterhin ist in Hinblick auf Fig. 3 zu erläutern, dass am Beinauflageabschnitt 70 eine Federeinrichtung 90 vorgesehen ist. Diese Federeinrichtung 90 ist in Form einer Schrauben-Zugfeder gestaltet, deren erster Anlenkpunkt 92A unmittelbar am Beinauflagenabschnitt 70 vorgesehen ist. Der zweite Anlenkpunkt 92B ist an einem Federschieber 96 vorgesehen, der innerhalb einer Federspur 94 des Beinauflagenabschnitts 70 verschiebbar ist. Die Relativbewegung der Schwenkhebel 60, 62 zum Beinauflagenabschnitt 70 ist dadurch hergestellt, dass zwischen dem Schwenkhebel 60 und dem Federschieber 92 eine an beiden schwenkbeweglich angelenkte Federlasche 98 wirkt.

In Fig. 3 ist gestrichelt die Beinauflageeinheit 170 dargestellt. Es ist ersichtlich, dass am hinteren Ende eine zentrale schlitzartige Unterbrechung 176 vorgesehen ist, die sich in Möbellängsrichtung (X) erstreckt. Diese Unterbrechung 176 dient der Aufnahme der Schwenkhebel 60, 62 bei Anordnung der Beinauflageneinheit 170 in der Stauposition.

Die Beinauflageeinheit 170 verfügt in nicht näher dargestellter Weise über eine Tragplatte, die gemeinsam mit einem Polstermaterial von einem Bezug umgeben ist. Dieser Bezug ist vorzugsweise an seiner hinteren Seite 178 offen und mittels eines Reißverschlusses oder eines anderweitigen Mechanismus verschließbar. Diese offene Seite gestattet es, den Bezug mitsamt der Tragplatte und dem Polstermaterial von vorne auf den Beinauflagenabschnitt 70 aufzuschieben. Vorzugsweise wird dabei eine formschlüssige Kopplung mit dem Beinauflagenabschnitt 70 werkzeuglos durch Verrasten odgl. hergestellt.

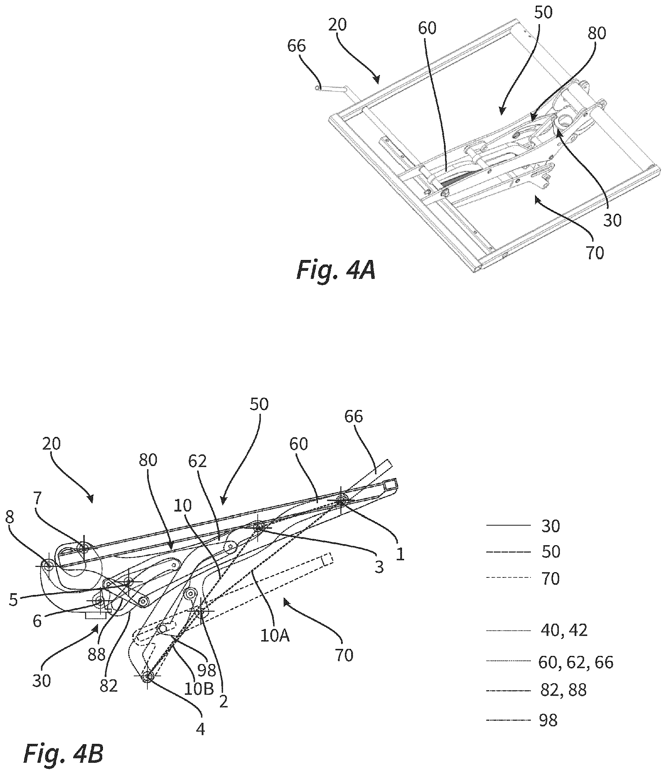

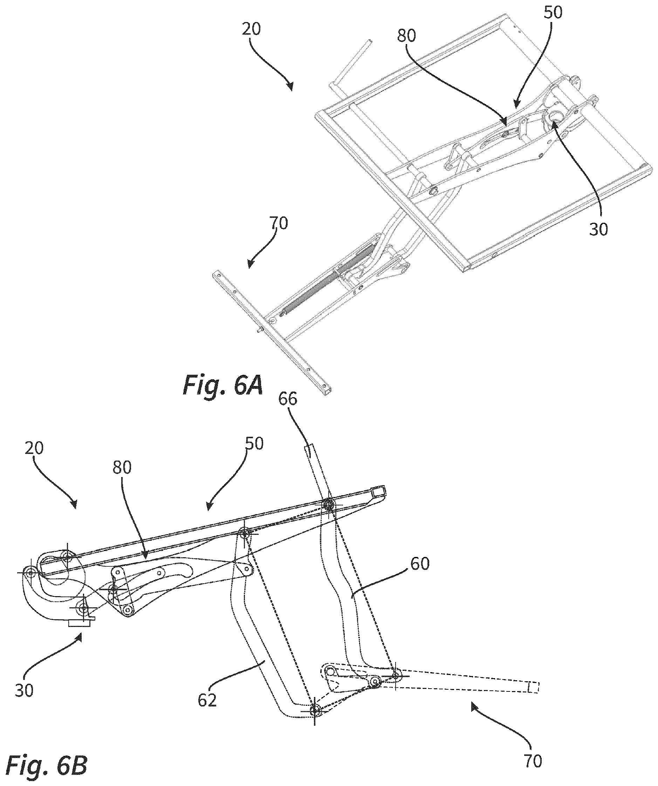

Anhand der Fig. 4A bis 7B wird nachfolgend die Funktionsweise des Möbelbeschlags 20 beim Ausfahren des Beinauflagenabschnitts 70 erläutert.

Fig. 4A und 4B zeigen in Entsprechung zur Fig. 1 einen Ausgangszustand, in dem der Beinauflagenabschnitt 70 unterhalb des Sitzflächenabschnitts 50 angeordnet ist. Durch den Linienzug wird ein Viereck 10 verdeutlicht, welches durch die Schwenkachsen 1, 2, 3, 4 aufgespannt wird. Wie anhand der Fig. 4B ersichtlich ist, ist dieses Viereck im Ausgangszustand in einer sogenannten Übertotpunktlage und daher in Art eines konvexen Vierecks geformt. Die zweite Schwenkachse 2 ist also jenseits einer Verbindungslinie zwischen erster und dritter Schwenkachse 1, 3 angeordnet.

Eine auf die Sitzflächeneinheit 150 wirkende Gewichtskraft der auf dem Sitzmöbelstück sitzenden Person wirkt sich in dieser Ausgangslage noch nicht dahingehend aus, dass der Beinauflagenabschnitt 70 in Richtung seiner Nutzposition kraftbeaufschlagt wird, da zwar die Gewichtskraft die beiden Schwenklaschen 40, 42 im Uhrzeigersinn bezogen auf Fig. 4B momentenbeaufschlagt, das Führungselement 86 am Ende des Auslegers 44 der Schwenklasche 42 ist jedoch aufgrund der Ausrichtung der Zwischenlasche 88 noch nicht in der Lage, ein gegen den Uhrzeigersinn gerichtetes Moment in die Schwenkhebel 62, 60 des Beinauflagenabschnitts 70 einzukoppeln.

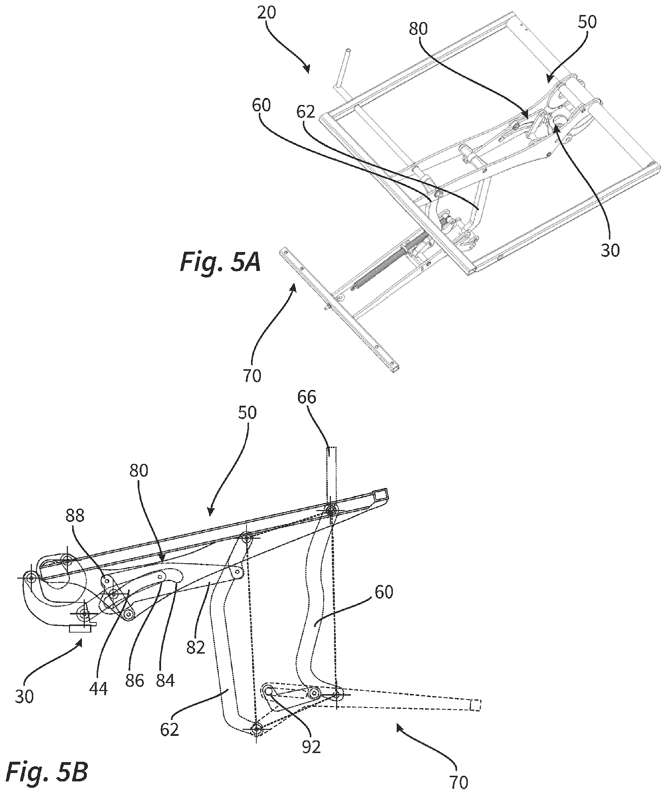

Um die Überführung zu initiieren, bedarf es daher einer manuellen Momenteneinkopplung am Betätigungshebel 66. Hierdurch werden, wie der Übergang von den Fig. 4A, 4B zu den Fig. 5A, 5B zeigt, die beiden Schwenkhebel 60, 62 gegen den Uhrzeigersinn ausgelenkt, wobei auch die zuvor gegebene Übertotpunktlage aufgelöst wird. Aufgrund der Formgebung der Steuerspur 84 in der Antriebslasche 82 geht diese Bewegung zunächst nicht mit einer Bewegung des Führungselements 86 einher, so dass auch kein Verschwenken der Schwenklaschen 40, 42 und kein Absenken der Sitzflächeneinheit 150 stattfindet.

Erst wenn in etwa der Zustand der Fig. 5B erreicht ist, ist die Zwischenlasche 88 ausreichend weit verschwenkt, so dass die Gewichtskraft des auf dem Sitzmöbelstück Sitzenden ab nun beginnt, ein gegen den Uhrzeigersinn gerichtetes Drehmoment in die Schwenkhebel 60, 62 einzukoppeln. Somit kann eine weitere manuelle Beaufschlagung des Betätigungshebels 66 nun unterbleiben. Angetrieben durch die Gewichtskraft des auf dem Sitzmöbelstück Sitzenden wird die Beinauflagenabschnitt 70 weiter in Richtung ihrer Nutz-Endstellung getrieben.

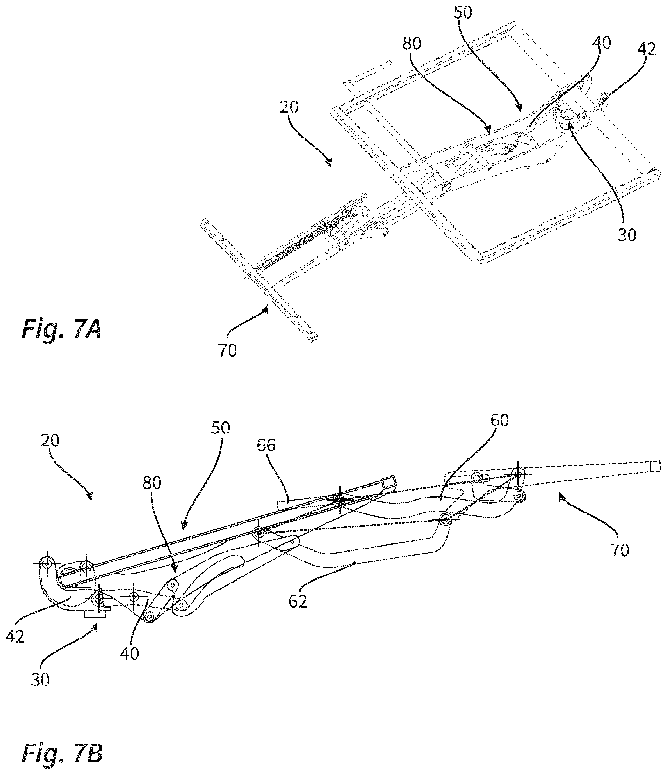

Der Zustand der Fig. 5A und 5B ist gleichzeitig auch der Umschlagzeitpunkt in Hinblick auf die Federeinrichtung 90, also der Zustand maximaler Spannung in der Federeinrichtung 90. Bei der fortgesetzten Bewegung in Richtung des Zustandes der Fig. 7A, 7B unterstützt nunmehr die Feder das weitere Anheben der Beinauflageeinheit 170, bis diese im Zustand der Fig. 7A und 7B ihre Nutzposition einnimmt. In dieser Nutz-Endlage ist die durch die Komponenten der Getriebeeinrichtung 80 bewirkte Übersetzung maximal, d.h. die Gewichtskraft des auf dem Sitzmöbelstück Sitzenden stabilisiert im Zustand der Fig. 7A, 7B die Position des Beinauflagenabschnitts 70.

Wird nun ausgehend vom Zustand der Fig. 7A, 7B eine Rücküberführung in den Ausgangszustand der Fig. 4A und 4B gewünscht, so erfolgt dies dadurch, dass ein im Uhrzeigersinn gerichtetes Moment durch den Sitzenden in dem Beinauflagenabschnitt 70 und somit in die Schwenkhebel 60, 62 eingekoppelt wird, wobei in haptisch angenehmer Weise die größte Kraft zu Beginn zu leisten ist. Ab dann geht die fortgesetzte Bewegung immer leichter vonstatten, was der Übersetzungscharakteristik des Getriebes geschuldet ist. Sobald bei der Rücküberführung wieder der Zustand der Fig. 5A und 5B erreicht ist, ist die Sitzflächeneinheit 150 in ihrer oberen Endlage, so dass die fortgesetzte Bewegung bis in den Zustand der Fig. 4A und 4B alleine durch die Federkraft der Federeinrichtung 90 und die beim Einklappen im Zustand der Fig. 5A und 5B vorhandene kinetische Energie des Beinauflagenabschnitts 70 bewirkt wird.

Wie anhand der Figuren gut zu ersehen ist, ist der Möbelbeschlag 20 ausgesprochen kompakt und mit Ausnahme des Torsionsrohres 65 und des Betätigungshebels 66 auch vollständig in einem nur maximal 15 cm breiten Bereich in der Mitte des Sitzmöbelstücks angeordnet. Der Möbelbeschlag 20 ist daher am Sitzmöbelstück nahezu unsichtbar. Dies ist auch der geringen Zahl von Teilen geschuldet, die erforderlich sind. Dies wiederum ist auch Ergebnis der Tatsache, dass der Beinauflagenabschnitt 70 und der Sitzflächenabschnitt 50 durch die beiden Schwenkhebel 60, 62 unmittelbar miteinander verbunden sind. Andere Möbelbeschläge sehen hier längere Ketten gegeneinander beweglicher Elemente vor, die demzufolge auch voluminöser im Aufbau sind.

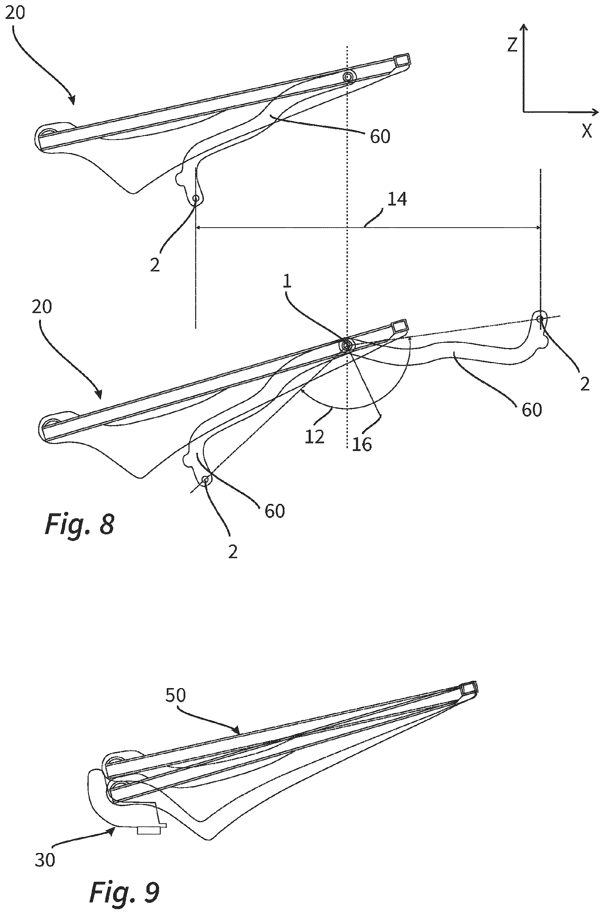

Anhand der Fig. 8 und 9 ist zu ersehen, wie groß die Bewegung des ersten Schwenkhebels ist und wie dieser mit der Bewegung des Sitzflächenabschnitts gekoppelt ist.

Wie sich der Fig. 8 ersehen lässt, wird durch den vergleichsweise langen Schwenkhebel 60 und insbesondere durch die Tatsache, dass der vordere Schwenkhebel um einen Schwenkwinkel 12 von mehr als 140° relativ zum Sitzflächenabschnitt verschwenkbar ist, ein weiter Ausfahrweg ermöglicht. Vorliegend wird die zweite Schwenkachse gegenüber der ersten Schwenkachse im Zuge der Überführung um eine Strecke 14 von über 50 cm in Möbellängsrichtung verlagert.

Fig. 9 verdeutlicht die Bewegung des Sitzflächenabschnitts 50 relativ zum Basisabschnitt 30. Die oberen Endlage (gestrichelt dargestellt) nimmt der Sitzflächenabschnitts 50 ein, bis der Schwenkhebel 60 fast die mit 16 in Fig. 8 gekennzeichnete Halblage erreicht hat. Erst bei der fortgesetzten Bewegung des Schwenkhebels 60 senkt sich der Sitzflächenabschnitts 50 bis in die nicht gestrichelt dargestellte Stellung in Fig. 9.

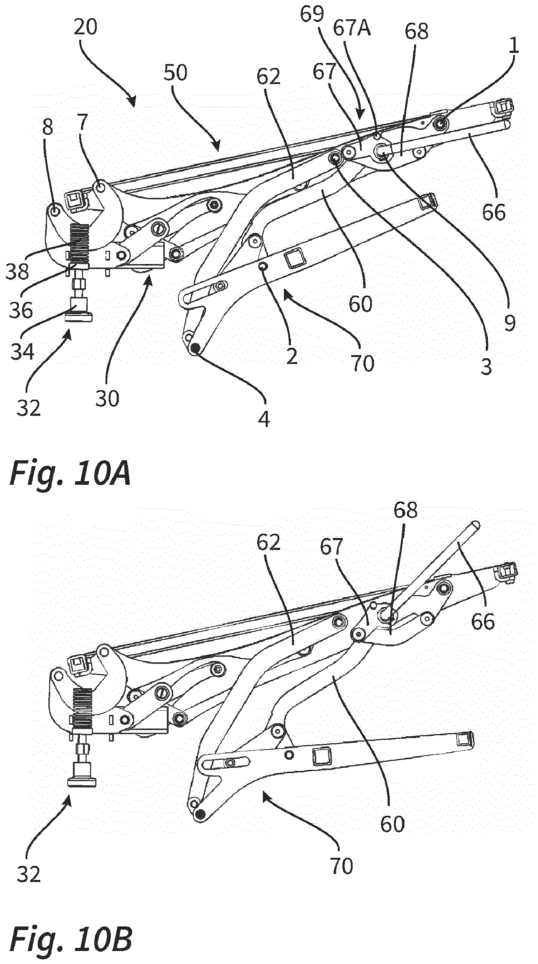

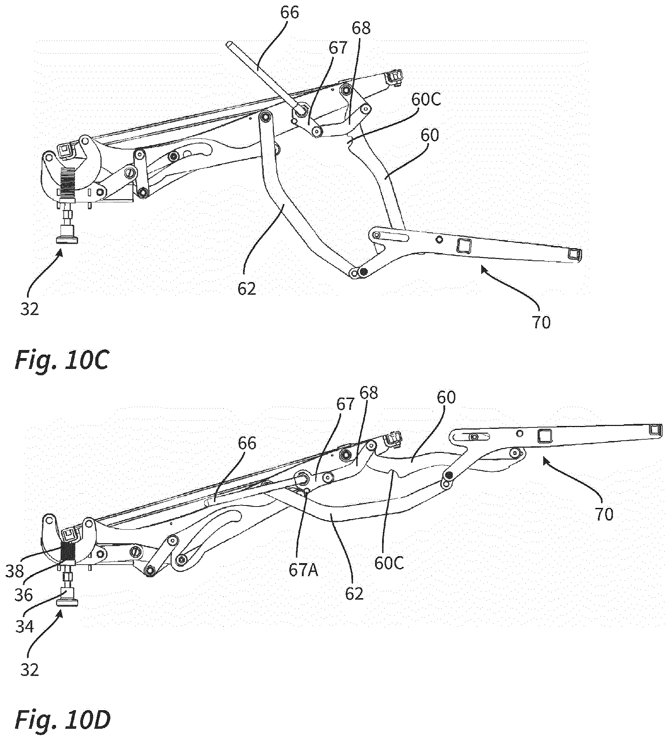

Das Ausführungsbeispiel der Fig. 10A bis 10D ähnelt dem vorangegangenen Ausführungsbeispiel weitgehend. Bis auf die im Folgenden erläuterten Unterschiede gelten alle obigen Ausführungen auch für dieses zweite Ausführungsbeispiel. Die Figuren zeigen das Ausfahren des Beinauflagesegmentes von der Stau-Endlage (Fig. 10A) bis zur Nutz-Endlage (Fig. 10D)

Der erste wesentliche Unterschied betrifft die Kopplung des Betätigungshebels 66 mit dem Schwenkhebel 60. Während beim obigen Ausführungsbeispiel hier eine feste Verbindung besteht, ist beim zweiten Ausführungsbeispiel ein Antriebsgetriebe 69 zwischengeschaltet. Der Betätigungshebel 66 ist wie auch der Schwenkhebel 60 schwenkbar am Sitzflächenabschnitt 50 angebracht, allerdings um eine von der Schwenkachse 1 des Schwenkhebels 60 beabstandete Hebelachse 9.

Die Koppelung des Betätigungshebels 66 an den Schwenkhebel 60 erfolgt über das Antriebsgetriebe 69, welches über eine Hebellasche 68 verfügt, die an einem drehfest mit dem Betätigungshebel 66 verbundenen Abschnitt 67 exzentrisch zur Hebelachse 9 schwenkbar angelenkt ist. Das gegenüberliegende Ende der Hebellasche 68 ist schwenkbar am Schwenkhebel 60 angelenkt. Wie der Übergang von Fig. 10A bis Fig. 10D zeigt, ist es hierdurch ebenfalls möglich, durch Verschwenken des Betätigungshebels 66 ein Antriebsmoment in den Schwenkhebel 60 einzukoppeln.

DerVorteil gegenüber der direkten Verbindung von Schwenkhebel 60 und Betätigungshebel 66 gemäß dem vorherigen Ausführungsbeispiel liegt darin, dass das Antriebsgetriebe eine variable Übersetzung bereitstellt. Zu Beginn der Überführung, also in der Phase, in der die Gewichtskraft des Benutzers kaum einen Beitrag zum Verschwenken des Beinauflageabschnitts 70 leistet, erleichtert die anfängliche Übersetzung des Betätigungshebels 66 von etwa 10:1 das Verschwenken des Betätigungshebels erheblich. Dieses hohe Übersetzungsverhältnis kommt zustande, da der Anlenkpunkt der Hebellasche 68 am Abschnitt 67 fast auf Höhe der Hebelachse 9 liegt und somit nur eine sehr geringe Verlagerung der Hebellasche 68 in Möbellängsrichtung stattfindet. Da diese in Möbellängsrichtung stattfindende Verlagerung der Hebellasche 68 jedoch für das Verschwenken des Schwenkhebels 60 zunächst verantwortlich ist, ergibt sich bei vergleichsweise starkem Verschwenken des Betätigungshebels 66 ein nur geringes Verschwenken des Schwenkhebels 60 mit entsprechend höherem Moment. Der Beinauflageabschnitt 70 kann somit ohne große Körperkraft aus der Stau-Endlage in Richtung der Nutz-Endlage verlagert werden. Durch das Verschwenken des Abschnitts 67 ändert sich zunehmen die Relativstellung des Anbindungspunktes der Hebellasche 68 am Abschnitt 67 relativ zur Hebelachse 9, so dass die Übersetzung bis etwa in den Bereich von 1:1 sinkt.

Beim vorliegenden Ausführungsbeispiel ist im Kontext mit dem Antriebsgetriebe 69 noch eine weitere Besonderheit realisiert. Am Abschnitt 67, der drehfest mit dem Betätigungshebel 66 verbunden ist, ist ein sich in Möbelquerrichtung erstreckender Stift 67A angebracht. Korrespondierend mit diesem ist am Schwenkhebel 60 eine seitliche Vertiefung 60C vorgesehen. An der in Fig. 10C nach unten weisende Flanke dieser Vertiefung 60C liegt der Stift 67A in der Stau-Endlage der Fig. 10A an. Dies bewirkt, dass mit Beginn der Überführung des Beinauflageabschnitts 70 aus der Stau-Endlage in Richtung der Nutz-Endlage die Kraftübertragung auf den Schwenkhebel 60 nicht nur über die Hebellasche 68, sondern auch über den Stift 67A und die Flanke der Vertiefung 60C erfolgt. Dies trägt der Tatsache Rechnung, dass ein solches Kniehebel-Konzept, wie es durch die Hebellasche 68 realisiert ist, aufgrund von Fertigungstoleranzen in der spitzwinkeligen Stellung der Fig. 10A zur Ungenauigkeit neigt. Es ist daher von Vorteil, dass die Kombination des Stiftes 67A und der Vertiefung 60C in dieser kritischen Phase ebenfalls die Kraftübertragung auf den Schwenkhebel 60 ermöglichen.

Der zweite wesentliche Unterschied zum ersten Ausführungsbeispiel liegt in der Federanordnung 32. Diese Federanordnung 32 umfasst eine Druckfeder 38, deren unteres Ende an einer Anlagefläche 36 anliegt. Diese wiederum ist mittels einer Drehhandhabe 34 und eines nicht näher dargestellten Gewindes höhenverstellbar gegenüber dem Basisabschnitt 30 an diesem angebracht.

Wie sich anhand der Figuren 10A bis 10C ersehen lässt, übernimmt die Federanordnung 32 während der Überführung zunächst keine Funktion. Erst im letzten Augenblick vor Erreichen der Nutz-Endlage, dargestellt in Fig. 10D, kommt eine hintere Strebe des Sitzflächenabschnitts 50 in Kontakt mit dem oberen Ende der Druckfeder 38 und komprimiert diese, bis sie mit Erreichen der Nutz-Endlage den maximal komprimierten Zustand aufweist.

Die Druckfeder 38 bewirkt dann eine auf den Sitzflächenabschnitt wirkende Kraft, die versucht, den Sitzflächenabschnitt nach oben zu drücken und somit den Beinauflageabschnitt 70 wieder in Richtung der Stau-Endlage kraftzubeaufschlagen. Das Niederdrücken des Beinauflageabschnitts 70 zum Zwecke der Rücküberführung des Beinauflageabschnitts 70 in die Stau-Endlage wird somit erleichtert.

Auf diese Weise kann das Sitzmöbelstück an die Körperproportionen einer Person angepasst werden. Je kürzer die Beine des Nutzers sind, desto schwerer fällt es ihm, die Rücküberführung über die Beine einzuleiten. Die Unterstützung durch die Druckfeder erleichtert es. Für Personen mit längeren Beinen kann die Anlagefläche 36 abgesenkt werden, so dass die Wirkung der Druckfeder 38 verringert wird oder gar vollständig entfällt.

## Patentansprüche

1. Möbelbeschlag (20) für ein Sitzmöbelstück (100) mit den folgenden Merkmalen:
a. der Möbelbeschlag (20) weist einen als in sich starres Bauteil ausgebildeten Basisabschnitt (30) zur Anbringung an einer auf einem Fußboden stehenden Basiseinheit (130) auf und
b. der Möbelbeschlag (20) weist einen als in sich starres Bauteil ausgebildeten Sitzflächenabschnitt (50) zur Anbringung einer Sitzflächeneinheit (150) mit Sitzfläche (152) auf und
c. der Möbelbeschlag (20) weist einen als in sich starres Bauteil ausgebildeten Beinauflagenabschnitt (70) zur Anbringung einer Beinauflageneinheit (170) mit Beinauflagefläche (172) auf und
d. der Sitzflächenabschnitt (50) ist beweglich zwischen einer oberen und einer unteren Endlage am Basisabschnitt (30) angebracht und
e. der Beinauflagenabschnitt (70) ist beweglich zwischen einer Stau-Endlage, in der der Beinauflageabschnitt (70) unter dem Sitzflächenabschnitt (50) angeordnet ist, und einer Nutz-Endlage, in der der Beinauflageabschnitt (70) vor der Sitzflächenabschnitt (50) angeordnet ist, am Sitzflächenabschnitt (50) angebracht und
f. der Beinauflageabschnitt (70) ist mittels eines hinteren und eines vorderen Schwenkhebels (60, 62) am Sitzflächenabschnitt (50) angebracht, wobei
- einer der Schwenkhebel (60) um eine zum Sitzflächenabschnitt und diesem Schwenkhebel (60) ortsfeste erste Schwenkachse (1) gegenüber dem Sitzflächenabschnitt (50) schwenkbeweglich ist und um eine zum Beinauflagenabschnitt (70) und diesem Schwenkhebel (60) ortsfeste zweite Schwenkachse gegenüber dem Beinauflagenabschnitt (70) schwenkbeweglich ist, und
- der andere Schwenkhebel (62) um eine zum Sitzflächenabschnitt (50) und diesem Schwenkhebel (62) ortsfeste dritte Schwenkachse (3) gegenüber dem Sitzflächenabschnitt (50) schwenkbeweglich ist und um eine zum Beinauflagenabschnitt (70) und diesem Schwenkhebel (62) ortsfeste vierte Schwenkachse (4) gegenüber dem Beinauflagenabschnitt (70) schwenkbeweglich ist, und
g. es ist eine Getriebeeinrichtung (80) vorgesehen zur zumindest phasenweisen kinematischen Kopplung der Bewegung des Sitzflächenabschnitts (50) gegenüber dem Basisabschnitt (30) mit der Bewegung des Beinauflagenabschnitts (70) gegenüber dem Sitzflächenabschnitt (50).

2. Möbelbeschlag (20) nach Anspruch 1 mit den folgenden Merkmalen:
a. der Möbelbeschlag weist eine Antriebseinrichtung (64) auf, mittels derer ein erster der Schwenkhebel (60) zum Zwecke des Überführen des Beinauflageabschnitts (70) in die Nutz-Endlage angetrieben ist, und
b. die Getriebeeinrichtung (80) weist eine Übersetzungscharakteristik auf, der zufolge bei der Überführung des Beinauflageabschnitts (70) aus der Stau-Endlage in die Nutz-Endlage
- in einer Bewegungsphase ausgehend von der Stau-Endlage, in der der erste Schwenkhebel (60) 50% seines Weges zwischen der Stau-Endlage und der Nutz-Endlage des Beinauflageabschnitts (70) zurücklegt, der Sitzflächenabschnitt (50) weniger als 50%, vorzugsweise weniger als 25%, insbesondere vorzugsweise weniger als 15% seines Weges zwischen der oberen Endlage und der unteren Endlage zurücklegt, und
- in einer sich anschließenden Bewegungsphase bis zur Nutz-Endlage, in der der erste Schwenkhebel (60) weitere 50% seines Weges zwischen der Stau-Endlage und der Nutz-Endlage des Beinauflageabschnitts (70) zurücklegt, der Sitzflächenabschnitt (50) mehr als 50% seines Weges zwischen der oberen Endlage und der unteren Endlage zurücklegt.

3. Möbelbeschlag (20) nach Anspruch 2 mit den folgenden Merkmalen:
a. die Getriebeeinrichtung (80) weist eine Übersetzungscharakteristik auf, der zufolge bei der Überführung des Beinauflageabschnitts (70) aus der Stau-Endlage in die Nutz-Endlage in einer Bewegungsphase ausgehend von der Stau-Endlage, in der der erste Schwenkhebel (60) 30% seines Weges zwischen der Stau-Endlage und der Nutzstellung des Beinauflageabschnitts (70) zurücklegt, der Sitzflächenabschnitt (50) weniger als 10%, vorzugsweise weniger als 5%, seines Weges zwischen der oberen Endlage und der unteren Endlage zurücklegt.

4. Möbelbeschlag (20) nach einem der vorstehenden Ansprüche mit dem folgenden Merkmal
a. der Sitzflächenabschnitt (50) ist mittels mindestens einer Schwenklasche (40) an den Basisabschnitt (30) angebunden, und
b. an einem der Schwenkhebel (62) ist eine Antriebslasche (82) schwenkbar angelenkt und
c. die Getriebeeinrichtung (80) ist zwischen der Schwenklasche (40) und der Antriebslasche (82) vorgesehen und verfügt über eine Steuerspur (84), entlang derer ein von der Schwenklasche (40) angetriebenes Führungselement (86) beweglich ist.
vorzugsweise mit den zusätzlichen Merkmalen:
a. das Führungselement (86) ist ortsfest an der Schwenklasche (40) vorgesehen und
b. die Steuerspur (84) weist eine gekrümmte Formgebung auf.

5. Möbelbeschlag (20) nach einem der Ansprüche 2 bis 4 mit einem der folgenden Merkmale:
a. die Antriebseinrichtung (64) umfasst einen Betätigungshebel (66) zur manuellen Betätigung, der drehfest mit einem der Schwenkhebel (60) verbunden ist, oder
b. die Antriebseinrichtung umfasst einen Elektromotor, der in einen der Schwenkhebel ein Antriebsmoment einkoppelt.

6. Möbelbeschlag (20) nach einem der Ansprüche 1 bis 4 mit dem folgenden Merkmal:
a. die Antriebseinrichtung umfasst ein Betätigungselement (66) zur manuellen Betätigung, welches über ein Antriebsgetriebe (69) in einen der Schwenkhebel (60) ein Antriebsmoment einkoppelt,
vorzugsweise mit den folgenden zusätzlichen Merkmalen:
b. das Betätigungselement (66) ist als schwenkbarer Betätigungshebel (66) ausgebildet und
c. der Betätigungshebel (66) ist über eine Hebellasche (68), die schwenkbar an einem mit dem Betätigungshebel (66) drehfesten Abschnitt (67) und schwenkbar mit dem Schwenkhebel (60) verbunden ist, am Schwenkhebel (60) angebracht.

7. Möbelbeschlag nach einem der vorstehenden Ansprüche mit den folgenden Merkmalen:
a. in der Stau-Endlage schließt eine Verbindungslinie (10A) zwischen der ersten Schwenkachse (1) und der zweiten Schwenkachse (2) mit einer Verbindungslinie (10B) zwischen der zweiten Schwenkachse (2) und der vierten Schwenkachse (4) einen Winkel < 20° ein, und/oder
b. in der Stau-Endlage befindet sich die zweite Schwenkachse (2) in einer ÜbertotpunktLage, so dass ein durch die Lage der ersten, zweiten, dritten und vierten Schwenkachse (1, 2, 3, 4) definiertes Viereck (10) ein konkaves Viereck (10) bildet, dessen zweite Schwenkachse (2) die konkave Ecke (11) bildet.

8. Möbelbeschlag (20) nach einem der vorstehenden Ansprüche, **gekennzeichnet durch** das folgende Merkmal:
a. ein Schwenkwinkel (12), den einer der Schwenkhebel (60) zwischen der Stau-Endlage und der Nutz-Endlage des Beinauflageabschnittes (70) gegenüber dem Sitzflächenabschnitt (50) überstreicht, beträgt mindestens 130°, vorzugsweise mindestens 140°, vorzugsweise mit dem zusätzlichen Merkmal:
b. ein Schwenkwinkel, den der andere Schwenkhebel (62) zwischen der Stau-Endlage und der Nutz-Endlage des Beinauflageabschnittes (70) gegenüber dem Sitzflächenabschnitt (50) überstreicht, beträgt mindestens 110°, vorzugsweise mindestens 120°,

9. Möbelbeschlag (20) nach einem der vorstehenden Ansprüche, **gekennzeichnet durch** mindestens eines der folgenden Merkmale:
a. der vordere Schwenkhebel (60) ist um die erste Schwenkachse (1) gegenüber dem Sitzflächenabschnitt schwenkbeweglich und um die zweite Schwenkachse (2) gegenüber dem Beinauflagenabschnitt schwenkbeweglich, und/oder
b. der Schwenkwinkel (12) des vorderen Schwenkhebels (60), den der vordere Schwenkhebel (60) zwischen der Stau-Endlage und der Nutz-Endlage des Beinauflageabschnittes (70) überstreicht, beträgt mindestens 130°, vorzugsweise mindestens 140°, und/oder
c. der Betätigungshebel (66) der Antriebseinrichtung (64) ist drehfest mit dem vorderen Schwenkhebel (60) verbunden, und/oder
d. die Antriebslasche (82) ist am hinteren Schwenkhebel (62) schwenkbar angelenkt.

10. Möbelbeschlag (20) nach einem der vorstehenden Ansprüche mit dem Merkmal:
a. der Basisabschnitt (30) und der Sitzflächenabschnitt (50) sind über zwei Schwenklaschen (40, 42) miteinander verbunden, wobei
- eine erste Schwenklasche (40) um eine zur ersten Schwenklasche (40) und zum Sitzflächenabschnitt (50) ortsfeste fünfte Schwenkachse (5) schwenkbar am Sitzflächenabschnitt (50) angebracht ist und um eine zur ersten Schwenklasche (40) und zum Basisabschnitt (30) ortsfeste sechste Schwenkachse (6) schwenkbar am Basisabschnitt (30) angebracht ist, und
- eine zweite Schwenklasche (42) um eine von der fünften Schwenkachse (5) abweichende und zur zweiten Schwenklasche (42) und zum Sitzflächenabschnitt (50) ortsfeste siebente Schwenkachse (7) schwenkbar am Sitzflächenabschnitt (50) angebracht ist und um eine von der sechsten Schwenkachse (6) abweichende und zur zweiten Schwenklasche (42) und zum Basisabschnitt (30) ortsfeste achte Schwenkachse (8) schwenkbar am Basisabschnitt (30) angebracht ist.
vorzugsweise mit dem zusätzlichen Merkmal:
a. die fünfte und die siebente Schwenkachse (5, 7) sind voneinander um nicht mehr als 25 cm, vorzugsweise um nicht mehr als 15 cm, beabstandet und vorzugsweise in einem rückwärtigen Bereich des Möbelbeschlages (20) vorgesehen.

11. Möbelbeschlag (20) nach einem der vorstehenden Ansprüche mit den folgenden Merkmalen:
a. der Möbelbeschlag (20) verfügt über eine Federeinrichtung (90), mittels derer der Sitzflächenabschnitt (50) und der Beinauflagenabschnitt (70) gegeneinander federkraftbeaufschlagt werden, und
b. die Federeinrichtung (90) ist am Beinauflagenabschnitt (70) vorgesehen.

12. Möbelbeschlag (20) nach Anspruch 11 mit dem folgenden Merkmal:
a. der Beinauflageabschnitt (70) definiert eine Haupterstreckungsebene der daran anzubringenden Beinauflageeinheit (170), und
b. die Federeinrichtung (90) ist als Federeinrichtung mit zwei entlang einer Federrichtung linear gegeneinander beweglichen Anlenkpunkten (92A, 92B) ausgebildet, wobei die Federrichtung in der Haupterstreckungsebene des Beinauflageabschnitts (70) liegt oder mit dieser einen Winkel von weniger als 20° einschließt,
vorzugsweise mit den zusätzlichen Merkmalen:
c. die Federeinrichtung (90) ist an zwei Anlenkpunkten (92A, 92B) an unterschiedlichen Komponenten des Möbelbeschlags angebunden,
d. ein erster Anlenkpunkt (92A) ist am Beinauflagenabschnitt (70) vorgesehen,
e. ein zweiter Anlenkpunkt (92B) ist an einem der Schwenkhebel vorgesehen oder an einem entlang einer Federspur (94) verschieblichen Federschieber (96) vorgesehen, der über einer Federlasche (98) mit einem der Schwenkhebel (60) gekoppelt ist.

13. Möbelbeschlag (20) nach einem der vorstehenden Ansprüche mit dem folgenden Merkmal:
a. es ist eine Federanordnung (32) vorgesehen, die eine Feder (38) umfasst, welche den Sitzflächenabschnitt (50) in jenem Zustand, in dem der Beinauflageabschnitt (70) seine Nutz-Endlage erreicht hat, nach oben oder nach unten kraftbeaufschlagt, vorzugsweise mit dem zusätzlichen Merkmal:
b. die Federanordnung (32) ist manuell verstellbar, so dass die Kraft, mit der der Sitzflächenabschnitt (50) bei Erreichen der Nutz-Endlage des Beinauflageabschnitts (70) durch die Feder (38) beaufschlagt wird, einstellbar ist.

14. Möbelbeschlag (20) nach einem der vorstehenden Ansprüche mit mindestens einem der folgenden Merkmale:
a. es ist jeweils nur ein vorderer und ein hinterer Schwenkhebel (60, 62) vorgesehen, wobei die Schwenkhebel (60, 62) jeweils vorzugsweise schmaler als 3 cm sind, wobei insbesondere vorzugsweise an beiden Schwenkhebeln (60, 62) jeweils die erste, zweite, dritte und vierte Schwenkachse (1, 2, 3, 4) definierende Schwenkwellen (60A, 60B, 62A, 62B) fest an den Schwenkhebeln (60, 62) angebracht sind, oder
b. es sind mindestens zwei vordere Schwenkhebel und/oder mindestens zwei hintere Schwenkhebel vorgesehen, wobei die äußerer Schwenkhebel jeweils um nicht mehr als 15 cm in einer Möbelquerrichtung voneinander beabstandet sind, und/oder
c. es sind jeweils zwei erste Schwenklaschen (40) und zweite Schwenklaschen (42) vorgesehen, wobei die jeweils zwei Schwenklaschen (40, 42) um nicht mehr als 15 cm in einer Möbelquerrichtung (Y) voneinander beabstandet sind, und/oder
d. die Schwenkhebel (60, 62) sind derart am Sitzflächenabschnitt (50) und am Beinauflagenabschnitt (70) angebracht und weisen eine derartige Länge auf, dass der Beinauflagenabschnitt (70) zwischen seiner Stau-Endlage und seiner Nutz-Endlage gegenüber dem Sitzflächenabschnitt (50) zwischen 20° und 45° verschwenkt wird und/oder die zweite Schwenkachse (2) gegenüber der ersten Schwenkachse (1) um mindestens 40 cm in einer Möbellängsrichtung nach vorne verlagert wird, und/oder
e. die ersten und/oder die dritte Schwenkachse (1, 3) sind in Möbelhochrichtung (Z) unterhalb von Anbringungspunkten zur Anbringung der Sitzflächeneinheit angeordnet und/oder
f. die fünfte und/oder die siebte Schwenkachse (5, 7) sind in Möbelhochrichtung (Z) unterhalb von Anbringungspunkten zur Anbringung der Sitzflächeneinheit angeordnet und/oder
g. mindestens eine Schwenklasche (40, 42) ist bei Anordnung des Sitzflächenabschnitts in seiner unteren Endlage derart ausgerichtet, dass eine Verbindungslinie zwischen ihren Schwenkachsen (5, 6, 7, 8) mit der Möbellängsrichtung einen Winkel <30°, insbesondere <20° einschließt, und/oder
h. die sechste und die achte Schwenkachse (6, 8) sind am Basisabschnitt (30) bezogen auf die Möbelhochrichtung (Z) auf unterschiedlichen Höhen vorgesehen und vorzugsweise in Möbelhochrichtung (Z) um mindestens 4 cm voneinander beabstandet, und/oder
i. der Möbelbeschlag umfasst einen Rückenlehnenabschnitt zur Anbringung einer Rückenlehne, wobei der Rückenlehnenabschnitt vorzugsweise fest mit dem Sitzflächenabschnitt verbunden ist oder mittels eines Getriebes derart mit dem Basisabschnitt und die Sitzflächenabschnitt verbunden ist, dass er sich in Abhängigkeit der Relativstellung des Sitzflächenabschnitts gegenüber dem Basisabschnitt gegenüber beiden verlagert und/oder
j. die vierte Schwenkachse (4) ist an einem nach unten ragenden Fortsatz des Beinauflageabschnitts (30 vorgesehen.

15. Sitzmöbelstück (100) in Art eines Sessels oder Sofas mit den folgenden Merkmalen:
a. das Sitzmöbelstück (100) verfügt über einen Möbelbeschlag (20) nach einem der vorstehenden Ansprüche,
b. das Sitzmöbelstück (100) verfügt über eine Basiseinheit (130), an der der Basisabschnitt (30) des Möbelbeschlages (20) angebracht ist,
c. das Sitzmöbelstück verfügt über eine Sitzflächeneinheit (150), die eine Sitzfläche (152) des Sitzmöbelstücks (100) aufweist und die an dem Sitzflächenabschnitt (50) des Möbelbeschlages (20) angebracht ist, und
d. das Sitzmöbelstück (100) verfügt über eine Beinauflageneinheit (170), die eine Beinauflagenfläche (172) aufweist und die am Beinauflagenabschnitt (70) des Möbelbeschlages (20) angebracht ist.

16. Sitzmöbelstück (100) nach Anspruch 15 mit mindestens einem der folgenden Merkmale:
a. die Beinauflagenfläche (172) weist mindestens eine bezogen auf die Nutzstellung der Beinauflageneinheit in Richtung der Sitzflächeneinheit weisende Unterbrechung (176) auf, in die mindestens einer der Schwenkhebel (60, 62) eintaucht, wenn sich die Beinauflageneinheit (170) in ihrer Stau-Endstellung befindet, wobei vorzugsweise ein Bereich der Beinauflagenfläche (172), in dem die mindestens eine zur Aufnahme des mindestens einen Schwenkhebels (60, 62) vorgesehene Unterbrechung (176) vorgesehen ist, bezogen auf eine Möbelquerrichtung (Y) zentrisch angeordnet ist und eine Breite von maximal 15 cm weist auf und/oder
b. die Basiseinheit weist mindestens einen Möbelstückfuß zum Aufstellen des Möbelstücks auf, an dem ortsfest der Basisabschnitts des Möbelbeschlages angebracht ist, oder
c. die Basiseinheit (130) ist als drehbare Basiseinheit (130) ausgebildet mit mindestens einem Möbelstückfuß (132) zum Aufstellen des Möbelstücks, an dem um eine Vertikalachse drehbar ein Drehteil (134) vorgesehen ist, an dem ortsfest oder einstückig der Basisabschnitts (30) des Möbelbeschlages (20) angebracht ist, und/oder
d. das Sitzmöbelstück (100) ist als Sessel ausgebildet oder
e. das Sitzmöbelstück ist als Sofa ausgebildet, wobei vorzugsweise mindestens zwei Beschläge nach einem der Ansprüche 1 bis 15 vorgesehen sind, durch die mindestens zwei Sitzflächeneinheiten und zwei Beinauflageeinheiten unabhängig voneinander gegenüber einer gemeinsamen Basiseinheit verlagerbar sind.
f. die ersten und/oder die dritte Schwenkachse (1, 3) sind in Möbelhochrichtung (Z) unterhalb der Sitzfläche (152) angeordnet, vorzugsweise um mindestens 2 cm, insbesondere vorzugsweise um mindestens 4 cm, und/oder
g. die fünfte und/oder die siebte Schwenkachse (5, 7) sind in Möbelhochrichtung (Z) unterhalb der Sitzfläche (152) angeordnet, vorzugsweise um mindestens 2 cm, insbesondere vorzugsweise um mindestens 4 cm, und/oder
h. die Sitzflächeneinheit verfügt am vorderen Ende unterhalb der Sitzfläche über eine Blende, wobei eine Vorderkante der Beinauflageneinheit in der Stauposition bezogen auf die Möbellängsrichtung (X) hinter diese Blende angeordnet ist und/oder
i. die Beinauflageeinheit (170) verfügt über einen Bezug, der eine Polsterung und einen Träger umgibt, wobei der Bezug an seinem rückwärtigen Ende (178) offen ist, um von vorne auf den Beinauflagenabschnitt (70) aufgeschoben zu werden, wobei vorzugsweise ein Beinauflageabschnitt (70) einerseits und am Träger der Beinauflageeinheit Kopplungsabschnitte sind, die im Zuge des Aufschiebens miteinander werkzeuglos eine formschlüssig wirkende Verrastung bewirken, und/oder
j. die vierte Schwenkachse (4) ist an einem nach unten ragenden Fortsatz des Beinauflageabschnitts (30 vorgesehen und mindestens 5 cm unterhalb der Beinauflagefläche (172) angeordnet, vorzugsweise mindestens 7 cm.

## Claims

1. Furniture fitting (20) for an item of seating furniture (100), having the following features:
a. the furniture fitting (20) has a base portion (30) in the form of an inherently rigid component for attachment to a base unit (130) that stands on a floor, and
b. the furniture fitting (20) has a seat face portion (50) in the form of an inherently rigid component for attaching a seat face unit (150) having a seat face (152), and
c. the furniture fitting (20) has a leg rest portion (70) in the form of an inherently rigid component for attaching a leg rest unit (170) having a leg rest face (172), and
d. the seat face portion (50) is attached to the base portion (30) such that it can be moved between an upper and a lower end position, and
e. the leg rest portion (70) is attached to the seat face portion (50) such that it can be moved between a stowage end position, in which the leg rest portion (70) is arranged under the seat face portion (50), and a use end position, in which the leg rest portion (70) is arranged in front of the seat face portion (50), and
f. the leg rest portion (70) is attached to the seat face portion (50) by means of a rear and a front pivoting lever (60, 62), wherein
- one of the pivoting levers (60) is pivotably movable with respect to the seat face portion (50) about a first pivot axis (1), which is stationary with respect to the seat face portion and with respect to said pivoting lever (60), and is pivotably movable with respect to the leg rest portion (70) about a second pivot axis, which is stationary with respect to the leg rest portion (70) and with respect to said pivoting lever (60), and
- the other pivoting lever (62) is pivotably movable with respect to the seat face portion (50) about a third pivot axis (3), which is stationary with respect to the seat face portion (50) and with respect to said pivoting lever (62), and is pivotably movable with respect to the leg rest portion (70) about a fourth pivot axis (4), which is stationary with respect to the leg rest portion (70) and with respect to said pivoting lever (62), and
g. a gear device (80) is provided for the kinematic coupling at least in phases of the movement of the seat face portion (50) with respect to the base portion (30) with the movement of the leg rest portion (70) with respect to the seat face portion (50).

2. Furniture fitting (20) according to Claim 1, having the following features:
a. the furniture fitting has a drive device (64), by means of which a first one of the pivoting levers (60) is driven for the purpose of transferring the leg rest portion (70) into the use end position, and
b. the gear device (80) has a transmission characteristic, according to which, when the leg rest portion (70) is being transferred from the stowage end position into the use end position,
- in a movement phase starting from the stowage end position, in which the first pivoting lever (60) covers 50% of its travel between the stowage end position and the use end position of the leg rest portion (70), the seat face portion (50) covers less than 50%, preferably less than 25%, particularly preferably less than 15% of its travel between the upper end position and the lower end position, and,
- in a subsequent movement phase up to the use end position, in which the first pivoting lever (60) covers a further 50% of its travel between the stowage end position and the use end position of the leg rest portion (70), the seat face portion (50) covers more than 50% of its travel between the upper end position and the lower end position.

3. Furniture fitting (20) according to Claim 2, having the following feature:
a. the gear device (80) has a transmission characteristic, according to which, when the leg rest portion (70) is being transferred from the stowage end position into the use end position, in a movement phase starting from the stowage end position, in which the first pivoting lever (60) covers 30% of its travel between the stowage end position and the use position of the leg rest portion (70), the seat face portion (50) covers less than 10%, preferably less than 5%, of its travel between the upper end position and the lower end position.

4. Furniture fitting (20) according to one of the preceding claims, having the following features:
a. the seat face portion (50) is linked to the base portion (30) by means of at least one pivoting bracket (40), and
b. a drive bracket (82) is articulated pivotably on one of the pivoting levers (62), and
c. the gear device (80) is provided between the pivoting bracket (40) and the drive bracket (82), and has a control track (84), along which a guide element (86) which is driven by the pivoting bracket (40) can be moved,
preferably having the following additional features:
a. the guide element (86) is provided in a stationary manner on the pivoting bracket (40), and
b. the control track (84) has a curved shape.

5. Furniture fitting (20) according to one of Claims 2 to 4, having one of the following features:
a. the drive device (64) comprises an actuating lever (66) for manual actuation, which actuating lever is connected fixedly to one of the pivoting levers (60) for rotation therewith, or
b. the drive device comprises an electric motor which couples a drive torque into one of the pivoting levers.

6. Furniture fitting (20) according to one of Claims 1 to 4, having the following feature:
a. the drive device comprises an actuating element (66) for manual actuation, which actuating element couples a drive torque into one of the pivoting levers (60) via a drive gear mechanism (69),
preferably having the following additional features:
b. the actuating element (66) is in the form of a pivotable actuating lever (66), and
c. the actuating lever (66) is attached to the pivoting lever (60) via a lever bracket (68), which can be pivoted on a portion (67) which is fixed to the activation lever (66) for rotation therewith, and is pivotably connected to the pivoting lever (60).

7. Furniture fitting according to one of the preceding claims, having the following features:
a. in the stowage end position, a connecting line (10A) between the first pivot axis (1) and the second pivot axis (2) forms an angle of < 20° with a connecting line (10B) between the second pivot axis (2) and the fourth pivot axis (4), and/or,
b. in the stowage end position, the second pivot axis (2) is situated at a position beyond the top dead centre, with the result that a quadrilateral (10) which is defined by the position of the first, second, third and fourth pivot axes (1, 2, 3, 4) forms a concave quadrilateral (10), the second pivot axis (2) of which forms the concave corner (11).

8. Furniture fitting (20) according to one of the preceding claims, **characterized by** the following feature:
a. a pivoting angle (12) which one of the pivoting levers (60) sweeps over between the stowage end position and the use end position of the leg rest portion (70) with respect to the seat face portion (50) is at least 130°, preferably at least 140°,
preferably having the following additional feature:
b. a pivoting angle which the other pivoting lever (62) sweeps over between the stowage end position and the use end position of the leg rest portion (70) with respect to the seat face portion (50) is at least 110°, preferably at least 120°.

9. Furniture fitting (20) according to one of the preceding claims, **characterized by** at least one of the following features:
a. the front pivoting lever (60) can be moved pivotably about the first pivot axis (1) with respect to the seat face portion and can be moved pivotably about the second pivot axis (2) with respect to the leg rest portion, and/or
b. the pivoting angle (12) of the front pivoting lever (60) which the front pivoting lever (60) sweeps over between the stowage end position and the use end position of the leg rest portion (70) is at least 130°, preferably at least 140°, and/or
c. the actuating lever (66) of the drive device (64) is connected fixedly to the front pivot lever (60) for rotation therewith, and/or
d. the drive bracket (82) is articulated pivotably on the rear pivoting lever (62).

10. Furniture fitting (20) according to one of the preceding claims, having the following feature:
a. the base portion (30) and the seat face portion (50) are connected to one another via two pivoting brackets (40, 42), wherein
- a first pivoting bracket (40) is attached to the seat face portion (50) such that it can be pivoted about a fifth pivot axis (5), which is stationary with respect to the first pivoting bracket (40) and with respect to the seat face portion (50), and is attached to the base portion (30) such that it can be pivoted about a sixth pivot axis (6), which is stationary with respect to the first pivoting bracket (40) and with respect to the base portion (30), and
- a second pivoting bracket (42) is attached to the seat face portion (50) such that it can be pivoted about a seventh pivot axis (7), which differs from the fifth pivot axis (5) and is stationary with respect to the second pivoting bracket (42) and with respect to the seat face portion (50), and is attached to the base portion (30) such that it can be pivoted about an eighth pivot axis (8), which differs from the sixth pivot axis (6) and is stationary with respect to the second pivoting bracket (42) and with respect to the base portion (30),
preferably with the following additional feature:
a. the fifth and the seventh pivot axes (5, 7) are spaced apart from one another by not more than 25 cm, preferably by not more than 15 cm, and are preferably provided in a rear region of the furniture fitting (20).

11. Furniture fitting (20) according to one of the preceding claims, having the following features:
a. the furniture fitting (20) has a spring device (90), by means of which the seat face portion (50) and the leg rest portion (70) are loaded with a spring force counter to one another, and
b. the spring device (90) is provided on the leg rest portion (70).

12. Furniture fitting (20) according to Claim 11, having the following features:
a. the leg rest portion (70) defines a main plane of extent of the leg rest unit (170) that is to be attached thereto, and
b. the spring device (90) is in the form of a spring device with two articulation points (92A, 92B) which can be moved with respect to one another linearly along a spring direction, wherein the spring direction lies in the main plane of extent of the leg rest portion (70), or forms an angle of less than 20° with it,
preferably having the following additional features:
c. the spring device (90) is linked to different components of the furniture fitting at two articulation points (92A, 92B),
d. a first articulation point (92A) is provided on the leg rest portion (70),
e. a second articulation point (92B) is provided on one of the pivoting levers or is provided on a spring slide (96) which can be displaced along a spring track (94) and is coupled via a spring bracket (98) to one of the pivoting levers (60).

13. Furniture fitting (20) according to one of the preceding claims, having the following feature:
a. what is provided is a spring arrangement (32) comprising a spring (38) which, in that state in which the leg rest portion (70) has reached its use end position, loads the seat face portion (50) with an upwards or downwards force, preferably having the following additional feature:
b. the spring arrangement (32) can be manually adjusted so as to be able to set the force with which the seat face portion (50) is loaded by the spring (38) when the use end position of the leg rest portion (70) is reached.

14. Furniture fitting (20) according to one of the preceding claims, having at least one of the following features:
a. in each case only one front and one rear pivoting lever (60, 62) are provided, wherein the pivoting levers (60, 62) are in each case preferably narrower than 3 cm, wherein in each case pivoting shafts (60A, 60B, 62A, 62B) which define the first, second, third, and fourth pivot axes (1, 2, 3, 4) are attached fixedly to the pivoting levers (60, 62), in particular, preferably, on the two pivoting levers (60, 62), or
b. at least two front pivoting levers and/or at least two rear pivoting levers are provided, wherein the outer pivoting levers are spaced apart from one another in a furniture transverse direction in each case by not more than 15 cm, and/or
c. in each case two first pivoting brackets (40) and second pivoting brackets (42) are provided, wherein the respective two pivoting brackets (40, 42) are spaced apart from one another in a furniture transverse direction (Y) by not more than 15 cm, and/or
d. the pivoting levers (60, 62) are attached to the seat face portion (50) and to the leg rest portion (70) in such a way and have such a length that the leg rest portion (70) is pivoted by between 20° and 45° between its stowage end position and its use end position with respect to the seat face portion (50) and/or the second pivot axis (2) is shifted forwards in a furniture longitudinal direction with respect to the first pivot axis (1) by at least 40 cm, and/or
e. the first and/or the third pivot axis (1, 3) are/is arranged below attachment points for attaching the seat face unit in the furniture vertical direction (Z), and/or
f. the fifth and/or the seventh pivot axis (5, 7) are/is arranged below attachment points for attaching the seat face unit in the furniture vertical direction (Z), and/or,
g. when the seat face portion is arranged in its lower end position, at least one pivoting bracket (40, 42) is aligned in such a way that a connecting line between its pivot axes (5, 6, 7, 8) forms an angle of < 30°, in particular of < 20°, with the furniture longitudinal direction, and/or
h. the sixth and the eighth pivot axis (6, 8) are provided at different heights on the base portion (30) in relation to the furniture vertical direction (Z), and are preferably spaced apart from one another in the furniture vertical direction (Z) by at least 4 cm, and/or
i. the furniture fitting comprises a backrest portion for attaching a backrest, wherein the backrest portion preferably is connected fixedly to the seat face portion or is connected to the base portion and the seat face portion by means of a gear mechanism in such a way that it moves with respect to said seat face portion and said base portion depending on the relative position of the seat face portion with respect to the base portion, and/or
j. the fourth pivot axis (4) is provided on a downwardly protruding projection of the leg rest portion (30).

15. Item of seating furniture (100) in the manner of a chair or a sofa, having the following features:
a. the item of seating furniture (100) has a furniture fitting (20) according to one of the preceding claims,
b. the item of seating furniture (100) has a base unit (130), to which the base portion (30) of the furniture fitting (20) is attached,
c. the item of seating furniture has a seat face unit (150) which has a seat face (152) of the item of seating furniture (100) and is attached to the seat face portion (50) of the furniture fitting (20), and
d. the item of seating furniture (100) has a leg rest unit (170) which has a leg rest face (172) and is attached to the leg rest portion (70) of the furniture fitting (20).

16. Item of seating furniture (100) according to Claim 15, having at least one of the following features:
a. the leg rest face (172) has at least one interruption (176) which points in the direction of the seat face unit in relation to the use position of the leg rest unit and into which at least one of the pivoting levers (60, 62) dips when the leg rest unit (170) is situated in its stowage end position, wherein preferably a region of the leg rest face (172), in which region the at least one interruption (176) which is provided for receiving the at least one pivoting lever (60, 62) is provided, is arranged centrally in relation to a furniture transverse direction (Y) and has a width of at most 15 cm, and/or
b. the base unit has at least one furniture item base for setting up the item of furniture, to which furniture item base the base portion of the furniture fitting is attached in a stationary manner, or
c. the base unit (130) is in the form of a rotatable base unit (130) with at least one furniture item base (132) for setting up the item of furniture, on which furniture item base a rotary part (134) is provided such that it can be rotated about a vertical axis, to which rotary part the base portion (30) of the furniture fitting (20) is attached in a stationary or integral manner, and/or
d. the item of seating furniture (100) is in the form of a chair, or
e. the item of seating furniture is in the form of a sofa, wherein at least two fittings according to one of Claims 1 to 15 are preferably provided, by way of which at least two seat face units and two leg rest units can be moved with respect to a common base unit independently of one another,
f. the first and/or the third pivot axis (1, 3) are/is arranged below the seat face (152) in the furniture vertical direction (Z), preferably by at least 2 cm, particularly preferably by at least 4 cm, and/or
g. the fifth and/or the seventh pivot axis (5, 7) are/is arranged below the seat face (152) in the furniture vertical direction (Z), preferably by at least 2 cm, particularly preferably by at least 4 cm, and/or
h. the seat face unit has a cover at the front end below the seat face, wherein a front edge of the leg rest unit is arranged in the stowage position behind said cover in relation to the furniture longitudinal direction (X), and/or
i. the leg rest unit (170) has a covering which surrounds upholstery and a support, wherein the covering is open at its rear end (178) in order to be pushed onto the leg rest portion (70) from the front, wherein preferably there are coupling portions on the leg rest portion (70) firstly and on the support of the leg rest unit, which coupling portions bring about a mutual latching action, which acts in a positively locking manner, without tools while the pushing on is being carried out, and/or
j. the fourth pivot axis (4) is provided on a downwardly protruding projection of the leg rest portion (30) and is arranged at least 5 cm, preferably at least 7 cm, below the leg rest face (172) .

## Revendications

1. Ferrure de meuble (20) pour un meuble d'assise (100), comprenant les caractéristiques suivantes :
a. la ferrure de meuble (20) présente une portion de base (30) réalisée sous forme de composant rigide en soi pour le montage sur une unité de base (130) posée sur un sol et
b. la ferrure de meuble (20) présente une portion de surface de siège (50) réalisée sous forme de composant rigide en soi pour le montage d'une unité de surface de siège (150) avec une surface de siège (152) et
c. la ferrure de meuble (20) présente une portion de support de pied (70) réalisée sous forme de composant rigide en soi pour le montage d'une unité de support de pied (170) avec une surface de support de pied (172) et
d. la portion de surface de siège (50) est montée de manière déplaçable entre une position d'extrémité supérieure et une position d'extrémité inférieure sur la portion de base (30) et
e. la portion de support de pied (70) est montée sur la portion de surface de siège (50) de manière déplaçable entre une position d'extrémité de rangement, dans laquelle la portion de support de pied (70) est disposée en dessous de la portion de surface de siège (50), et une position d'extrémité d'utilisation, dans laquelle la portion de support de pied (70) est disposée devant la portion de surface de siège (50), et
f. la portion de support de pied (70) est montée sur la portion de surface de siège (50) au moyen d'un levier pivotant arrière et d'un levier pivotant avant (60, 62),
- l'un des leviers pivotants (60) pouvant être déplacé par rapport à la portion de surface de siège (50) par pivotement autour d'un premier axe de pivotement (1) fixe par rapport à la portion de surface de siège et à ce levier pivotant (60), et pouvant être déplacé par rapport à la portion de support de pied (70) par pivotement autour d'un deuxième axe de pivotement fixe par rapport à la portion de support de pied (70) et à ce levier pivotant (60), et
- l'autre levier pivotant (62) étant déplaçable par rapport à la portion de surface de siège (50) par pivotement autour d'un troisième axe de pivotement (3) fixe par rapport à la portion de surface de siège (50) et à ce levier pivotant (62) et étant déplaçable par rapport à la portion de support de pied (70) par pivotement autour d'un quatrième axe de pivotement (4) fixe par rapport à la portion de support de pied (70) et à ce levier pivotant (62), et
g. il est prévu un dispositif de transmission (80) pour un accouplement cinématique au moins temporaire du mouvement de la portion de surface de siège (50) par rapport à la portion de base (30) et du mouvement de la portion de support de pied (70) par rapport à la portion de surface de siège (50).

2. Ferrure de meuble (20) selon la revendication 1, comprenant les caractéristiques suivantes :
a. la ferrure de meuble présente un dispositif d'entraînement (64) au moyen duquel un premier des leviers pivotants (60) est entraîné en vue de transférer la portion de support de pied (70) dans la position d'extrémité d'utilisation, et
b. le dispositif de transmission (80) présente une caractéristique de démultiplication selon laquelle, dans le cas du transfert de la portion de support de pied (70) de la position d'extrémité de rangement dans la position d'extrémité d'utilisation,
- dans une phase de déplacement à partir de la position d'extrémité de rangement, dans laquelle le premier levier pivotant (60) parcourt 50 % de sa course entre la position d'extrémité de rangement et la position d'extrémité d'utilisation de la portion de support de pied (70), la portion de surface de siège (50) parcourt moins de 50 %, de préférence moins de 25 %, en particulier de préférence moins de 15 % de sa course entre la position d'extrémité supérieure et la position d'extrémité inférieure, et
- dans une phase de déplacement suivante jusqu'à la position d'extrémité d'utilisation, dans laquelle le premier levier pivotant (60) parcourt encore 50 % de sa course entre la position d'extrémité de rangement et la position d'extrémité d'utilisation de la portion de support de pied (70), la portion de surface de siège (50) parcourt plus de 50 % de sa course entre la position d'extrémité supérieure et la position d'extrémité inférieure.

3. Ferrure de meuble (20) selon la revendication 2, comprenant la caractéristique suivante :
a. le dispositif de transmission (80) présente une caractéristique de démultiplication selon laquelle, dans le cas du transfert de la portion de support de pied (70) de la position d'extrémité de rangement dans la position d'extrémité d'utilisation, dans une phase de déplacement à partir de la position d'extrémité de rangement, dans laquelle le premier levier pivotant (60) parcourt 30 % de sa course entre la position d'extrémité de rangement et la position d'utilisation de la portion de support de pied (70), la portion de surface de siège (50) parcourt moins de 10 %, de préférence moins de 5 %, de sa course entre la position d'extrémité supérieure et la position d'extrémité inférieure.

4. Ferrure de meuble (20) selon l'une quelconque des revendications précédentes, comprenant les caractéristiques suivantes :
a. la portion de surface de siège (50) est reliée au moyen d'au moins une patte pivotante (40) à la portion de base (30), et
b. une patte d'entraînement (82) est articulée de manière pivotante au niveau de l'un des leviers pivotants (62), et
c. le dispositif de transmission (80) est prévu entre la patte pivotante (40) et la patte d'entraînement (82) et dispose d'une trace de commande (84) le long de laquelle un élément de guidage (86) entraîné par la patte pivotante (40) peut être déplacé,
de préférence comprenant les caractéristiques supplémentaires suivantes :
a. l'élément de guidage (86) est prévu de manière fixe sur la patte pivotante (40) et
b. la trace de commande (84) présente une forme courbe.

5. Ferrure de meuble (20) selon l'une quelconque des revendications 2 à 4, comprenant l'une des caractéristiques suivantes :
a. le dispositif d'entraînement (64) comprend un levier d'actionnement (66) pour l'actionnement manuel, qui est connecté de manière solidaire en rotation à l'un des leviers pivotants (60), ou
b. le dispositif d'entraînement comprend un moteur électrique qui introduit un couple d'entraînement dans l'un des leviers pivotants.

6. Ferrure de meuble (20) selon l'une quelconque des revendications 1 à 4, comprenant la caractéristique suivante :
a. le dispositif d'entraînement comprend un élément d'actionnement (66) pour l'actionnement manuel, lequel introduit un couple d'entraînement par le biais d'un engrenage d'entraînement (69) dans l'un des leviers pivotants (60),
de préférence comprenant les caractéristiques supplémentaires suivantes :
b. l'élément d'actionnement (66) est réalisé sous forme de levier d'actionnement (66) pivotant (40) et
c. le levier d'actionnement (66) est monté sur le levier pivotant (60) par le biais d'une patte de levier (68) qui est connectée de manière pivotante au niveau d'une portion (67) solidaire en rotation avec le levier d'actionnement (66) et de manière pivotante au levier pivotant (60).

7. Ferrure de meuble selon l'une quelconque des revendications précédentes, comprenant les caractéristiques suivantes :
a. dans la position d'extrémité de rangement, une ligne de connexion (10A) entre le premier axe de pivotement (1) et le deuxième axe de pivotement (2) forme, avec une ligne de connexion (10B) entre le deuxième axe de pivotement (2) et le quatrième axe de pivotement (4), un angle < 20°, et/ou
b. dans la position d'extrémité de rangement, le deuxième axe de pivotement (2) se trouve dans une position de point mort haut, de telle sorte qu'un quadrilatère (10) défini par la position des premier, deuxième, troisième et quatrième axes de pivotement (1, 2, 3, 4) forme un quadrilatère concave (10), dont le deuxième axe de pivotement (2) forme le coin concave (11).

8. Ferrure de meuble (20) selon l'une quelconque des revendications précédentes, **caractérisée par** la caractéristique suivante :
a. un angle de pivotement (12), qui est balayé par l'un des leviers pivotants (60) entre la position d'extrémité de rangement et la position d'extrémité d'utilisation de la portion de support de pied (70) par rapport à la portion de surface de siège (50), vaut au moins 130°, de préférence au moins 140°,
de préférence comprenant la caractéristique supplémentaire suivante :
b. un angle de pivotement qui est balayé par l'autre levier pivotant (62) entre la position d'extrémité de rangement et la position d'extrémité d'utilisation de la portion de support de pied (70) par rapport à la portion de surface de siège (50), vaut au moins 110°, de préférence au moins 120°.

9. Ferrure de meuble (20) selon l'une quelconque des revendications précédentes, **caractérisée par** au moins l'une des caractéristiques suivantes :
a. le levier pivotant avant (60) est déplaçable par rapport à la portion de surface de siège par pivotement autour du premier axe de pivotement (1) et est déplaçable par rapport à la portion de support de pied autour du deuxième axe de pivotement (2), et/ou
b. l'angle de pivotement (12) du levier pivotant avant (60), qui est balayé par le levier pivotant avant (60) entre la position d'extrémité de rangement et la position d'extrémité d'utilisation de la portion de support de pied (70), vaut au moins 130°, de préférence au moins 140°, et/ou
c. le levier d'actionnement (66) du dispositif d'entraînement (64) est connecté de manière solidaire en rotation au levier pivotant avant (60), et/ou
d. la patte d'entraînement (82) est articulée de manière pivotante au niveau du levier pivotant arrière (62).

10. Ferrure de meuble (20) selon l'une quelconque des revendications précédentes, comprenant la caractéristique suivante :
a. la portion de base (30) et la portion de surface de siège (50) sont connectées l'une à l'autre par le biais de deux pattes pivotantes (40, 42),
- une première patte pivotante (40) étant montée sur la portion de surface de siège (50) de manière à pouvoir pivoter autour d'un cinquième axe de pivotement (5) fixe par rapport à la première patte pivotante (40) et à la portion de surface de siège (50), et étant montée sur la portion de base (30) de manière à pouvoir pivoter autour d'un sixième axe de pivotement (6) fixe par rapport à la première patte pivotante (40) et à la portion de base (30), et
- une deuxième patte pivotante (42) étant montée sur la portion de surface de siège (50) de manière à pouvoir pivoter autour d'un septième axe de pivotement (7) s'écartant du cinquième axe de pivotement (5) et fixe par rapport à la deuxième patte de pivotement (42) et à la portion de surface de siège (50) et étant montée sur la portion de base (30) de manière à pouvoir pivoter autour d'un huitième axe de pivotement (8) s'écartant du sixième axe de pivotement (6) et fixe par rapport à la deuxième patte pivotante (42) et à la portion de base (30),
de préférence comprenant la caractéristique supplémentaire :
a. le cinquième et le septième axe de pivotement (5, 7) sont espacés l'un de l'autre de pas plus de 25 cm, de préférence de pas plus de 15 cm, et de préférence sont prévus dans une région arrière de la ferrure de meuble (20).

11. Ferrure de meuble (20) selon l'une quelconque des revendications précédentes, comprenant les caractéristiques suivantes :
a. la ferrure de meuble (20) dispose d'un dispositif de ressort (90) au moyen duquel la portion de surface de siège (50) et la portion de support de pied (70) sont sollicitées l'une contre l'autre par la force du ressort, et
b. le dispositif de ressort (90) est prévu sur la portion de support de pied (70).

12. Ferrure de meuble (20) selon la revendication 11, comprenant les caractéristiques suivantes :
a. la portion de support de pied (70) définit un plan d'étendue principale de l'unité de support de pied (170) devant être montée sur celle-ci, et
b. le dispositif de ressort (90) est réalisé sous forme de dispositif de ressort avec deux points d'articulation (92A, 92B) déplaçables linéairement l'un par rapport à l'autre le long d'une direction de ressort, la direction de ressort étant située dans le plan d'étendue principale de la portion de support de pied (70) ou formant avec celle-ci un angle inférieur à 20°,
de préférence comprenant les caractéristiques supplémentaires suivantes :
c. le dispositif de ressort (90) est relié au niveau de deux points d'articulation (92A, 92B) à des composants différents de la ferrure de meuble,
d. un premier point d'articulation (92A) est prévu sur la portion de support de pied (70),
e. un deuxième point d'articulation (92B) est prévu au niveau de l'un des leviers pivotants ou au niveau d'un coulisseau à ressort (96) déplaçable le long d'une trace de ressort (94), qui est accouplé par le biais d'une patte élastique (98) à l'un des leviers pivotants (60).

13. Ferrure de meuble (20) selon l'une quelconque des revendications précédentes, comprenant la caractéristique suivante :
a. il est prévu un agencement de ressort (50) qui comporte un ressort (38), lequel sollicite par une force vers le haut ou vers le bas la portion de surface de siège (50) dans l'état dans lequel la portion de support de pied (70) a atteint sa position d'extrémité d'utilisation,
de préférence comprenant la caractéristique supplémentaire :
b. l'agencement de ressort (32) est déplaçable manuellement, de sorte que la force par laquelle la portion de surface de siège (50) est sollicitée par le ressort (38) lorsque la position d'extrémité d'utilisation de la portion de support de pied (70) est atteinte soit réglable.

14. Ferrure de meuble (20) selon l'une quelconque des revendications précédentes, comprenant au moins l'une des caractéristiques suivantes :
a. il est prévu à chaque fois seulement un levier pivotant avant et un levier pivotant arrière (60, 62), les leviers pivotants (60, 62) ayant chacun de préférence une largeur inférieure à 3 cm, en particulier de préférence au niveau des deux leviers pivotants (60, 62), des arbres de pivotement (60A, 60B, 62A, 62B) définissant à chaque fois à chaque fois les premier, deuxième, troisième et quatrième axes de pivotement (1, 2, 3, 4) étant montés fixement sur les leviers pivotants (60, 62), ou
b. il est prévu au moins deux leviers pivotants avant et/ou au moins deux leviers pivotants arrière, les leviers pivotants extérieurs n'étant à chaque fois pas espacés l'un de l'autre de plus de 15 cm dans une direction transversale du meuble et/ou
c. il est prévu à chaque fois deux premières pattes pivotantes (40) et deux deuxièmes pattes pivotantes (42), les deux pattes pivotantes respectives (40, 42) n'étant pas espacées l'une de l'autre de plus de 15 cm dans une direction transversale du meuble (Y) et/ou
d. les leviers pivotants (60, 62) sont montés de telle sorte sur la portion de surface de siège (50) et sur la portion de support de pied (70) et présentent une longueur telle que la portion de support de pied (70), entre sa position d'extrémité de rangement et sa position d'extrémité d'utilisation, soit pivotée par rapport à la portion de surface de siège (50) entre 20° et 45° et/ou que le deuxième axe de pivotement (2) soit décalé vers l'avant dans une direction longitudinale du meuble d'au moins 40 cm par rapport au premier axe de pivotement (1), et/ou
e. le premier et/ou le troisième axe de pivotement (1, 3) sont disposés dans la direction verticale du meuble (Z) en dessous de points de montage pour le montage de l'unité de surface de siège, et/ou
f. le cinquième et/ou le septième axe de pivotement (5, 7) sont disposés dans la direction verticale du meuble (Z) en dessous de points de montage pour le montage de l'unité de surface de siège et/ou
g. au moins une patte pivotante (40, 42) est orientée, lors de l'agencement de la portion de surface de siège dans sa position d'extrémité inférieure, de telle sorte qu'une ligne de connexion entre ses axes de pivotement (5, 6, 7, 8) forme avec la direction longitudinale du meuble un angle < 30°, en particulier < 20°, et/ou
h. le sixième et le huitième axe de pivotement (6, 8) sont prévus à différentes hauteurs sur la portion de base (30) par rapport à la direction verticale du meuble (Z) et de préférence sont espacés l'un de l'autre dans la direction verticale du meuble (Z) d'au moins 4 cm, et/ou
i. la ferrure de meuble comprend une portion de dossier pour le montage d'un dossier, la portion de dossier étant de préférence connectée fixement à la portion de surface de siège ou étant connectée au moyen d'une transmission à la portion de base et à la portion de surface de siège de telle sorte qu'elle se déplace, en fonction de la position relative de la portion de surface de siège par rapport à la portion de base, par rapport à ces portions et/ou
j. le quatrième axe de pivotement (4) est prévu au niveau d'une projection saillant vers le bas de la portion de support de pied (30).

15. Meuble d'assise (100) du type fauteuil ou sofa, comprenant les caractéristiques suivantes :
a. le meuble d'assise (100) dispose d'une ferrure de meuble (20) selon l'une quelconque des revendications précédentes,
b. le meuble d'assise (100) dispose d'une unité de base (130), sur laquelle est montée la portion de base (30) de la ferrure de meuble (20),
c. le meuble d'assise dispose d'une unité de surface de siège (150) qui présente une surface de siège (152) du meuble d'assise (100) et qui est montée sur la portion de surface de siège (50) de la ferrure de meuble (20), et
d. le meuble d'assise (100) dispose d'une unité de support de pied (170) qui présente une surface de support de pied (172) et qui est montée sur la portion de support de pied (70) de la ferrure de meuble (20) .

16. Meuble d'assise (100) selon la revendication 15, comprenant au moins l'une des caractéristiques suivantes:
a. la surface de support de pied (172) présente au moins une interruption (176) orientée dans la direction de l'unité de surface de siège par rapport à la position d'utilisation de l'unité de support de pied, dans laquelle est introduit au moins l'un des leviers pivotants (60, 62) lorsque l'unité de support de pied (170) se trouve dans sa position d'extrémité de rangement, une région de la surface de support de pied (172), dans laquelle est prévue l'au moins une interruption (176) prévue pour recevoir l'au moins un levier pivotant (60, 62), étant disposée de préférence centralement par rapport à une direction transversale du meuble (Y) et présentant une largeur maximale de 15 cm et/ou
b. l'unité de base présente au moins un pied de meuble pour poser le meuble, sur lequel est montée fixement la portion de base de la ferrure de meuble, ou
c. l'unité de base (130) est réalisée sous forme d'unité de base rotative (130), avec au moins un pied de meuble (132) pour poser le meuble, sur lequel est prévu une partie rotative (134) pouvant tourner autour d'un axe vertical, sur laquelle est monté fixement ou d'une seule pièce la portion de base (30) de la ferrure de meuble (20), et/ou
d. le meuble d'assise (100) est réalisé sous forme de fauteuil ou
e. le meuble d'assise est réalisé sous forme de sofa, au moins deux ferrures selon l'une quelconque des revendications 1 à 15 étant de préférence prévues, par le biais desquelles au moins deux unités de surface de siège et deux unités de support de pied peuvent être déplacées indépendamment l'une de l'autre par rapport à une unité de base commune,
f. le premier et/ou le troisième axe de pivotement (1, 3) sont disposés dans la direction verticale du meuble (Z) en dessous de la surface de siège (152), de préférence à au moins 2 cm, en particulier de préférence à au moins 4 cm, et/ou
g. le cinquième et/ou le septième axe de pivotement (5, 7) sont disposés dans la direction verticale du meuble (Z) en dessous de la surface de siège (152), de préférence à au moins 2 cm, en particulier de préférence à au moins 4 cm, et/ou
h. l'unité de surface de siège dispose d'un bandeau au niveau de l'extrémité avant en dessous de la surface de siège, une arête avant de l'unité de support de pied étant disposée dans la position de rangement derrière ce bandeau par rapport à la direction longitudinale du meuble (X) et/ou
i. l'unité de support de pied (170) dispose d'un revêtement qui entoure un rembourrage et un support, le revêtement étant ouvert au niveau de son extrémité arrière (178), afin de pouvoir être poussé depuis l'avant sur la portion de support de pied (70), de préférence des portions d'accouplement étant prévues sur la portion de support de pied (70) d'une part et sur le support de l'unité de support de pied, lesquelles au cours du mouvement de poussée provoquent ensemble, sans outil, un enclenchement agissant par engagement par correspondance de formes, et/ou
j. le quatrième axe de pivotement (4) est prévu au niveau d'une projection, saillant vers le bas, de la portion de support de pied (30), et est disposé à au moins 5 cm en dessous de la surface de support de pied (172), de préférence à au moins 7 cm.
